(19) **Europäisches Patentamt / European Patent Office / Office européen des brevets**

(11) **EP 1 897 484 B1**

(12) **EUROPEAN PATENT SPECIFICATION**

(45) Date of publication and mention
of the grant of the patent:
**30.12.2015 Bulletin 2015/53**

(51) Int Cl.:
*A61B 1/00* *(2006.01)*     *A61B 1/04* *(2006.01)*
*H04N 5/225* *(2006.01)*     *H04N 5/232* *(2006.01)*
*H04N 5/77* *(2006.01)*     *A61B 5/00* *(2006.01)*
*H04N 9/804* *(2006.01)*

(21) Application number: **07015701.1**

(22) Date of filing: **04.03.2004**

(54) **Endoscope image pickup device**

Bildaufnahmevorrichtung für ein Endoskop

Dispositif endoscopique de capture d'image

(84) Designated Contracting States:
**DE FR GB**

(43) Date of publication of application:
**12.03.2008 Bulletin 2008/11**

(62) Document number(s) of the earlier application(s) in
accordance with Art. 76 EPC:
**04717269.7 / 1 652 465**

(73) Proprietor: **Olympus Corporation
Tokyo 151-0072 (JP)**

(72) Inventors:
• **Homan, Masatoshi
Hachioji-shi
Tokyo 192-8512 (JP)**

• **Kotouda, Kaoru
Hachioji-shi
Tokyo 192-8512 (JP)**
• **Azuma, Motoo
Hachioji-shi
Tokyo 192-8512 (JP)**

(74) Representative: **von Hellfeld, Axel
Wuesthoff & Wuesthoff
Patentanwälte PartG mbB
Schweigerstrasse 2
81541 München (DE)**

(56) References cited:
**EP-A- 1 492 352**     **WO-A-03/010967**
**US-B1- 6 683 643**

EP 1 897 484 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to an endoscope image pick-up apparatus in which an image in the body is picked up by an image pick-up unit and the resultant image is transmitted by radio to an extra-corporeal unit.

Background Art

For example, Japanese Unexamined Patent Application Publication No. 2002-508201 discloses, as a conventional art, an endoscope image pick-up apparatus in which an image in the body is picked up by an image pick-up unit and the resultant image is transmitted by radio to an extra-corporeal unit.

According to the conventional art, the image pick-up unit, which is inserted in the body, incorporates an acceleration sensor as a movement detector in the axial direction. The acceleration sensor detects the movement in the axial direction, when the acceleration in the axial direction is lower than the preset threshold value, and a power source is shut off. Thus, the collection of redundant images is prevented and the consumption energy of the image pick-up unit is minimized.

However, the above conventional art needs to incorporate the acceleration sensor in the image pick-up unit and operates to shut off the power source by the acceleration sensor. There is a drawback in that a desired information cannot be properly obtained.

It is impossible to meet a requirement that the amount of images transmitted to the extra-corporeal unit from the image pick-up unit may be properly adjusted so as to transmit the amount of images in a target portion or an interest region without reducing the amount of images and to reduce the amount of images other than the target portion.

EP 1 492 352 A2 (D1) discloses an in-vivo imaging system comprising a device which may be implemented in a swallowable capsule and which includes an imager, an illumination source, and a transmitter. An image receiver, a storage unit, a data processor and a display unit are provided outside a patient's body. The transmitter may operate using radio waves and is adapted to transmit image data at a variable rate and/or using a variable format. In particular, image data captured by the imager is analyzed so as to determine whether the currently captured image is substantially similar and/or identical to a previously transmitted image. If it is determined that the currently captured image is similar and/or identical to a previously transmitted image, the currently captured image is not transmitted. Changes between subsequent images such as size, dimensions, light, color, brightness, contrast, borders, margins, focus, horizontal and/or vertical shifting may be determined. In another embodiment, one or several pixels of an image are compared, and differences in color properties, red-green-blue properties, hue saturation value properties, cyan-magenta-yellow properties, and cyan-magenta-yellow-black properties may be compared, and an image is transmitted if a pre-determined number of pixels in the second image are different from corresponding pixels of a first image. Further, color histograms may be compared and/or analyzed.

Therefore, the present invention is made above-described points. The object of the present invention is to provide an endoscope image pick-up unit which is capable of properly controlling the amount of images transmitted to the extra-corporeal unit without incorporating a sensor, e.g., the acceleration sensor in the image pick-up unit.

Disclosure of Invention

According to the present invention, there is provided an endoscope image pick-up apparatus according to claim 1. The endoscope image pick-up apparatus is adapted for picking up an image in the body by an image pick-up unit inserted in the body and for transmitting the image by radio to an extra-corporeal unit which is arranged outside the body, wherein the image pick-up unit comprises: image pick-up means for capturing an image; data transmitting means for transmitting the image obtained by the image pick-up means to the extra-corporeal unit at a plurality of transmitting rates; characteristic amount detecting means for detecting a predetermined amount of characteristics based on the image; and determining means for determining a valid image based on an output from the characteristic amount detecting means, and the data transmitting means controls the data transmitting rate in accordance with the determining result of the determining means. It is possible to control the amount of images transmitted to the extra-corporeal unit to the proper value without arranging the sensor in the image pick-up unit.

Brief Description of the Drawings

Figs. 1 to 19B relate to the first embodiment of the present invention, Fig. 1 is a schematic diagram showing a system according to the first embodiment;

Fig. 2 is a block diagram showing the schematic structure of an image pick-up unit;

Fig. 3 is a timing chart for the operation of the image pick-up unit;

Fig. 4 is a block diagram showing the structure of a processing block shown in Fig. 2;

Fig. 5 is a timing chart of the processing block;

Fig. 6 is a block diagram showing the structure of an invalid image detecting block shown in Fig. 4;

Fig. 7 is a block diagram showing the structure of a luminance range detecting block shown in Fig. 6;

Fig. 8 is a block diagram showing the structure of an image change detecting block shown in Fig. 6;

Fig. 9 is a block diagram showing the structure of an image change detecting block according to a modification;

Fig. 10 is a block diagram showing the structure of an affected part detecting block shown in Fig. 4;

Fig. 11 is a block diagram showing the structure of a specific color detecting block shown in Fig. 10;

Fig. 12 is a block diagram showing the structure of a specific-color change detecting block shown in Fig. 10;

Fig. 13 is a block diagram showing the structure of a color distribution characteristic detecting block shown in Fig. 10;

Figs. 14A to 14F are diagrams showing examples of the hue and saturation at a normal part and a color-changed part;

Fig. 15 is a block diagram showing the structure of a color space converting block;

Fig. 16 is a block diagram showing the structure of a hue histogram calculating block shown in Fig. 13;

Fig. 17A is an operation diagram of a histogram memory upon inputting a hue value A shown in Fig. 16;

Fig. 17B is an operation diagram of the histogram memory upon inputting a hue value B shown in Fig. 16;

Fig. 18 is a block diagram showing the structure of a hue distribution characteristic detecting block shown in Fig. 13;

Figs. 19A and 19B are explanatory diagrams showing the operation of the hue distribution characteristic detecting block;

Figs. 20 to 25 relate to the second embodiment of the present invention, Fig. 20 is a block diagram showing the structure of a processing block according to the second embodiment;

Fig. 21 is a timing chart of the processing block;

Fig. 22 is a block diagram showing the structure of an image size reducing block;

Fig. 23 is an explanatory diagram showing the operation of an image cutting-out block;

Fig. 24 is an explanatory diagram of the image reduction; and

Fig. 25 is a block diagram showing the structure of a compressing block.

Best Mode for Carrying Out the Invention

Hereinbelow, a description is given of embodiments of the present invention with reference to the drawings.

(First embodiment)

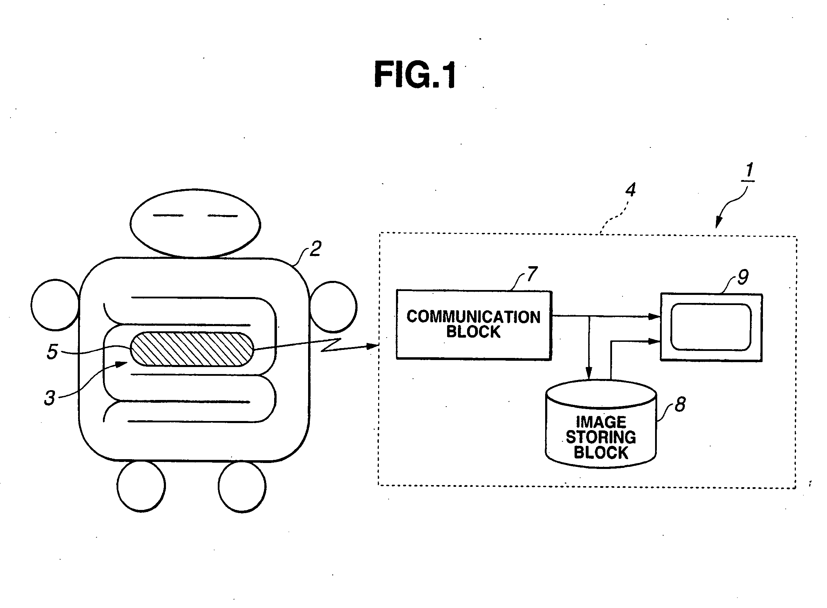

The first embodiment of the present invention will be described with reference to Figs. 1 to 19B. First, a description is given of the basic structure of a system according to the first embodiment with reference to Figs. 1 to 3.

Referring to Fig. 1, an endoscope image pick-up apparatus or endoscope image pick-up system 1 according to the first embodiment of the present invention comprises: an image pick-up unit 3 which is inserted in a body 2, then picks up an image of the body 2, and transmits image data thereof by radio; and an extra-corporeal unit 4 which receives the image data that is transmitted by radio from the image pick-up unit 3 and which stores and displays the image data.

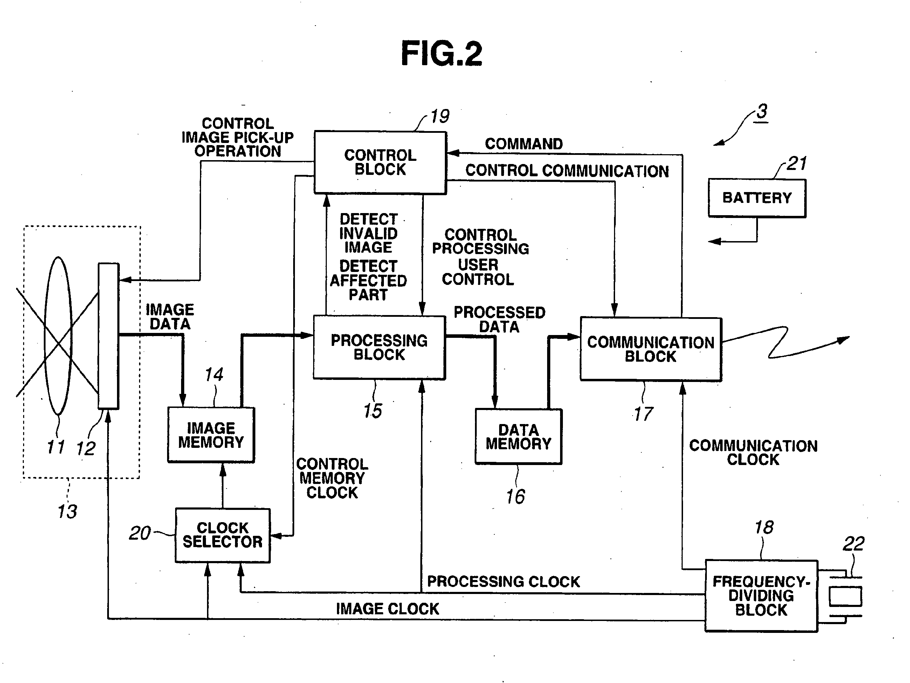

Referring to Fig. 1 again, the image pick-up unit 3 comprises a capsule sealed container 5 which includes: blocks having an image pick-up block 13, which will be described later with reference to Fig. 2; and a battery 21. The image pick-up unit 3 supplies electric energy from the battery 21 to the image pick-up block 13 or the like, and transmits by radio, to the extra-corporeal unit 4 arranged outside the body, the image picked-up by the image pick-up block 13.

The extra-corporeal unit 4 receives and demodulates the image data which is modulated and transmitted by radio from the image pick-up unit 3 from a communication block 7. The extra-corporeal unit 4 stores the demodulated image data to an image storing block 8, transmits the image data to a monitor 9, and displays the picked-up image onto a display surface of the monitor 9. The extra-corporeal unit 4 transmits the image data stored in the image storing block 8 to the monitor 9 side and displays the image.

Fig. 2 shows the structure of an electric system in the image pick-up unit 3. The image pick-up unit 3 comprises: the image pick-up block 13 comprising an objective optical system 11 for forming an optical image of an examination target part in the body cavity into which the image pick-up unit 3 is inserted and a (solid-state) image pick-up device 12 such as a CCD or CMOS sensor; an image memory 14 which temporarily stores, via an A/D converter (not shown), digital image data picked-up by the image pick-up device 12; a processing block 15 which performs various processing of the image data stored in the image memory 14; and a data memory 16 which temporarily stores the data processed by the processing block 15.

The image pick-up unit 3 comprises: a communication block 17 which reads the processed data from the data memory 16, transmits the read data to the extra-corporeal unit 4, and receives a command for controlling the image pick-up unit 3 from the extra-corporeal unit 4; and a frequency-dividing block 18 which generates clocks necessary for the blocks.

The image pick-up unit 3 further comprises: a control block 19 which outputs a control signal to the blocks; a clock selector 20 which changes the processing speed of the image data; the battery 21 which supplies power for driving the blocks and electric devices such as the image pick-up device 12; and an illuminating block having a white LED (not shown) for illuminating the examination target part picked-up by the image pick-up block 13.

The control block 19 outputs an image pick-up control signal for controlling the image pick-up operation, a processing control signal for controlling the processing, a communication control signal for controlling the communication, and a memory clock control signal for switching the frequency of an image clock for reading and writing data in the image memory 14, to the image pick-up device 12, the processing block 15, the communication block 17, and the clock selector 20, respectively.

The processing block 15 detects a predetermined characteristic amount of the image from the image memory 14, which will be described later. Upon determining that the image is a valid portion, e.g., an affected part (or target image) based on the output of the characteristic amount, the processing block 15 outputs an affected part detecting signal to the control block 19. Upon determining that the image is invalid, the processing block 15 outputs an invalid image detecting signal to the control block 19. When a command is received from the extra-corporeal unit 4, the communication block 17 supplies the command to the control block 19.

According to the first embodiment, for the purpose of the minimization of the image pick-up unit 3, clocks generated by a single crystal oscillator 22 are frequency-divided by the frequency diving block 18, and a communication clock supplied to the communication block 17, a processing clock supplied to the processing block 15, an image clock supplied to the image pick-up device 12 and image memory 14 are generated, respectively.

As will be described according to the second embodiment, a user transmits the command from the extra-corporeal unit 4 to the control block 19 and thus the control block 19 transmits, to the processing block 15, a user control signal as a control signal transmitted by the user. Then, the processing operation of the processing block 15 is controlled.

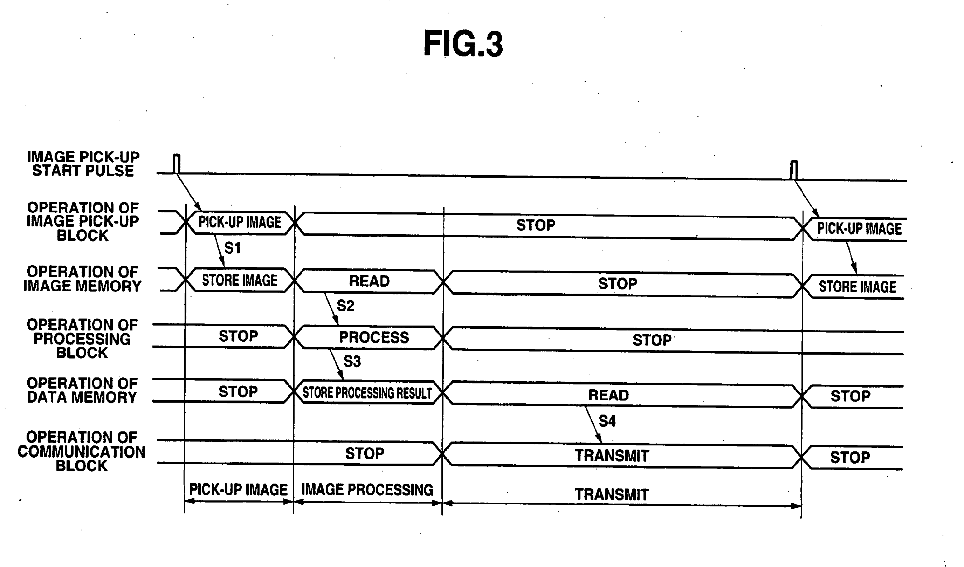

Fig. 3 shows a timing chart for the operation of the image pick-up unit 3.

An image pick-up start pulse is generated from a CPU (not shown) forming a controller of the control block 19 in order to transmit the image for a predetermined period. The image pick-up block 13 starts the image pick-up operation by the image pick-up start pulses and the picked-up image data is stored in the image memory 14 (this processing is shown by S1 in Fig. 3 and, similarly, the subsequent processing is shown by S2 and the like).

After storing the image data corresponding to one screen, the processing block 15 reads the image data from the image memory 14 (S2), performs the processing such as compression and characteristic detection, and stores the processing result thereof in the data memory 16 (S3). After ending the processing, the data stored in the data memory 16 is transmitted to the communication block 17, is modulated by the communication block 17, and is transmitted extra-corporeal unit 4 (S4).

In the basic system according to the first embodiment, referring to Fig. 3, the blocks have the sequence for preventing the simultaneous operation and thus the peak value of the consumption power from the battery 21 is reduced. That is, as shown by the bottom stage in Fig. 3, the sequence includes the sequential processing for time-dividing the image pick-up processing for picking up the image and storing the picked-up image, the imaging processing for reading the stored image, imaging the data, and storing the resultant data to the data memory 16, and the transmitting processing for reading the imaged data and transmitting the data.

As mentioned above, the image clock is supplied to the image memory 14 and the like.

The image signal needs a high-speed clock to some extent under the restriction of a frame rate.

Upon picking up the image, almost the blocks (internal block) are not operated, excluding the image pick-up block 13 in the image pick-up unit 3 shown in Fig. 3. Therefore, although the power consumption is not increased, almost the blocks in the image pick-up unit 3 are operated upon processing and transmitting the image signal. Thus, the power consumption is reduced by operating the internal blocks at a low-speed clock.

That is, the processing block 15 is operated at the processing clock speed lower than the image clock speed. Further, the communication block 17 is operated at the communication clock speed lower than the image clock speed.

In this case, the power consumption of the image memory 14 is reduced by using the clock selector 20 for switching the high-speed and low-speed clocks upon picking up and processing the image signal. That is, the control block 19 controls the clock selector 20 by a memory clock control signal, with shifting so as to supply a high speed image clock upon the picking up the image or a low speed processing clock upon processing the data to the image memory 14.

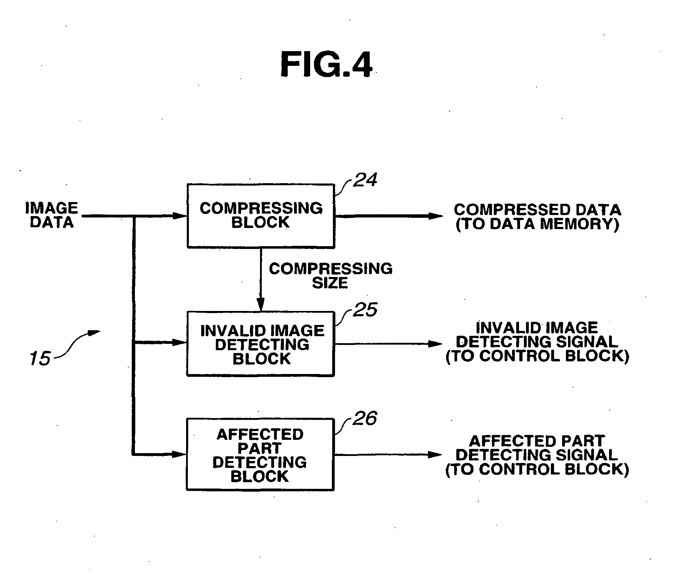

The detailed structure and operation will be described according to the first embodiment with reference to Figs. 4 to 19B. Fig. 4 shows the structure of the processing block 15 according to the first embodiment.

The image data inputted to the processing block is inputted to a compressing block 24 for compressing the image data, and an invalid image detecting block 25 and an affected part detecting block 26 forming characteristic amount detecting means for detecting the characteristic amount based on the image data and determining means for determining the validity.

The compressing block 24 forms compressed data which is obtained by reducing the data amount by compressing the image data. Then, the compressing block 24 stores the compressed data to the data memory 16.

The invalid image detecting block 25 detects the characteristic amount for the invalidity such as the white compression and black compression in the image and no change in image, and further determines whether or not the image is invalid. If it is detected that the image is invalid, the invalid image detecting block 25 outputs an image invalid detecting signal.

The affected part detecting block 26 detects the characteristic amount for the affected part or the present or absence of the similar matter in the image data, and determines based on the detecting result whether or not the image is a target image. If it is detected that the image is the target image, the affected part detecting block 26 outputs an affected part detecting signal.

The invalid image detecting signal and affected part detecting signal are inputted to the control block 19. The control block 19 controls the next image pick-up timing and the transmission of the image data based on the invalid image detecting signal and the affected part detecting signal.

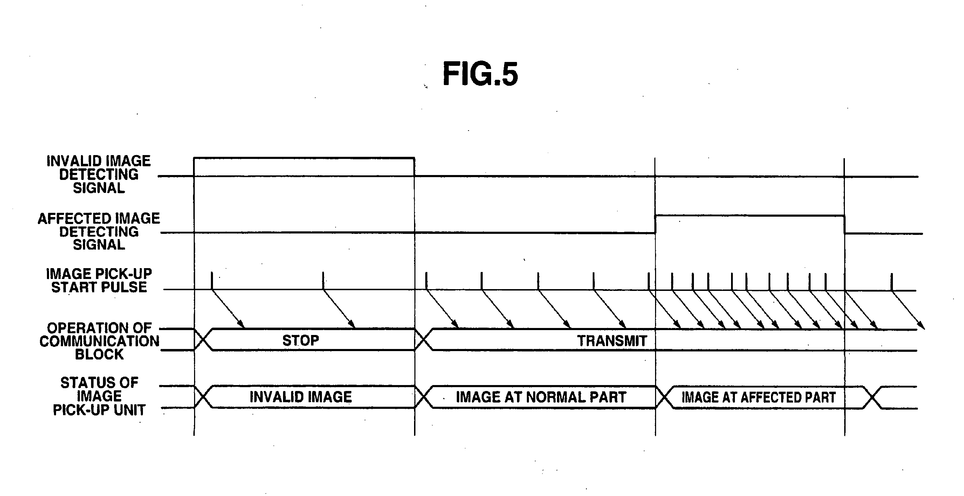

Fig. 5 shows a timing chart of the processing block 15.

First, when the invalid image detecting signal is active (H level), the processing block 15 does not transmit the image data and delays the next image pick-up period. Thus, the image which is determined as invalid and unnecessary is not subjected to the processing and the transmission (in this case, the status of the image pick-up unit 3 is referred to as an image invalid (status)).

When the invalid image detecting signal is not active and the affected part detecting signal is not active (H level), the image pick-up block 13 picks up the image for a predetermined period. The processing block 15 transmits the picked-up image as a normal-part image to the extra-corporeal unit 4 (in this case, the status of the image pick-up unit 3 is referred to as a normal-part image (status)).

Further, when the affected part detecting signal is active, it is considered that the target image is picked up. Therefore, the processing block 15 reduces the image pick-up and transmitting periods so as to improve the diagnosis capacity, obtains a large amount of images around the affected part, and transmits the obtained image to the extra-corporeal unit 4 (in this case, the status of the image pick-up unit 3 is referred to as an affected-part image (status)).

According to the first embodiment, the processing block 15 detects the characteristic amount of the picked-up image, determines whether or not the image is valid, that is, whether the characteristic amount includes an invalid portion or valid portion, and controls a transmitting rate of the image data transmitted to the extra-corporeal unit 4 from the communication block 17 in accordance with the determining result.

If it is determined that the image includes the invalid portion, the processing block 15 controls the transmitting rate to be stopped. If it is determined that the image is the normal image, the processing block 15 controls the transmitting rate to the normal transmitting rate. Further, if it is determined that the image includes the valid portion such as the affected part, the processing block 15 controls the transmitting rate to be increased.

As controlled above, the power of the battery 21 is consumed to transmit the image data with the large amount of information. The power consumption of the battery 21 is automatically adjusted in the proper state by increasing the transmitting rate of the valid image necessary for the user and then by reducing the transmitting rate of another image.

In addition to the above-mentioned operation, the control block 19 controls the image pick-up and transmitting periods by receiving the command from the extra-corporeal unit 4. Further, the control block 19 invalidates the control of the image pick-up and transmitting periods in the image pick-up unit 3 by receiving another command. The control operation based on the command from the extra-corporeal unit 4 is prior and the image pick-up and transmitting periods are controlled.

Thus, when the difficult determination is performed on the extra-corporeal unit 4, the determining result is transmitted to the image pick-up unit 3 by the command, and the image pick-up and transmitting rates of the image pick-up unit 3 is controlled by the command.

Next, a description is given of the detailed structure of the blocks and the operation thereof.

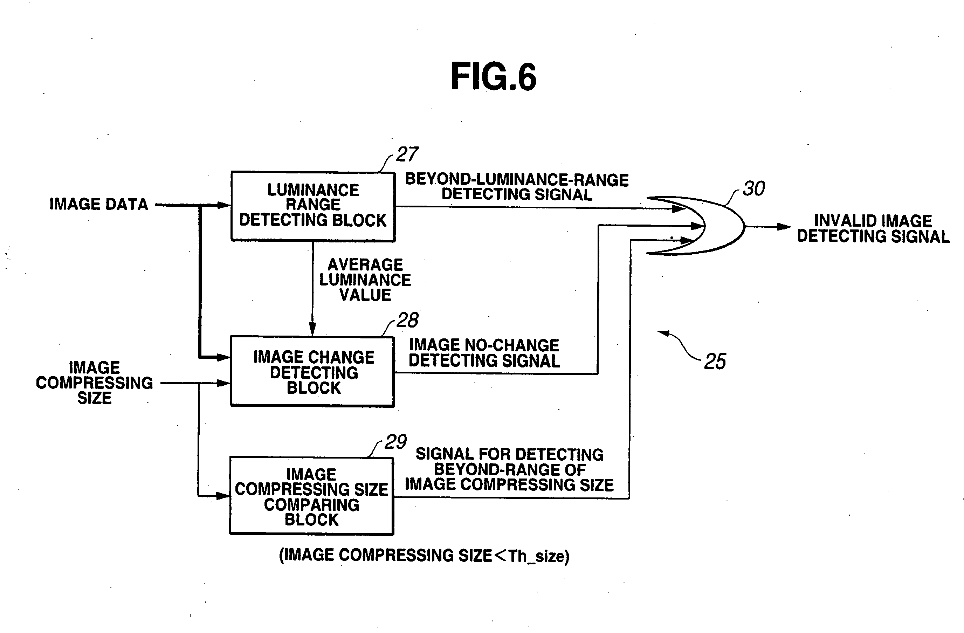

Fig. 6 shows the structure of the invalid image detecting block 25 shown in Fig. 4.

The invalid image detecting block 25 comprises: a luminance range detecting block 27; an image change detecting block 28; an image compressing size comparing block 29; and an OR circuit 30 to which the outputs signals therefrom are inputted.

The luminance range detecting block 27 detects the average value of the luminance value, and outputs, to the OR circuit 30, an beyond-luminance-range detecting signal when it is extremely bright or extremely dark. The image change detecting block 28 detects that the image does not change, that is, whether the image pick-up unit 3 does not move or moves in the body based on the image data, average luminance value, and (image) compressing size, and outputs an image non-change detecting signal to the OR circuit 30.

The image compressing size comparing block 29 compares the compressing size of image with a threshold value (Th_size). If it is determined that the compressing size of image is the threshold value (Th_size) or less, e.g., when the image is not focused with blur, the image compressing size comparing block 29 outputs, to the OR circuit 30, a signal for detecting the beyond-compressing-size of image. When any one of the beyond-luminance-range detecting signal, image non-change detecting signal and signal for detecting the beyond-compressing-size of image is detected, the invalid image detecting signal is outputted to the control block 19 via the OR circuit 30.

Referring to Fig. 6, symbols () denotes the operation for comparing the compressing size of image with the threshold value Th_size or less by the image compressing size comparing block 29 on the bottom. The foregoing comparing operation is similarly applied to another drawings.

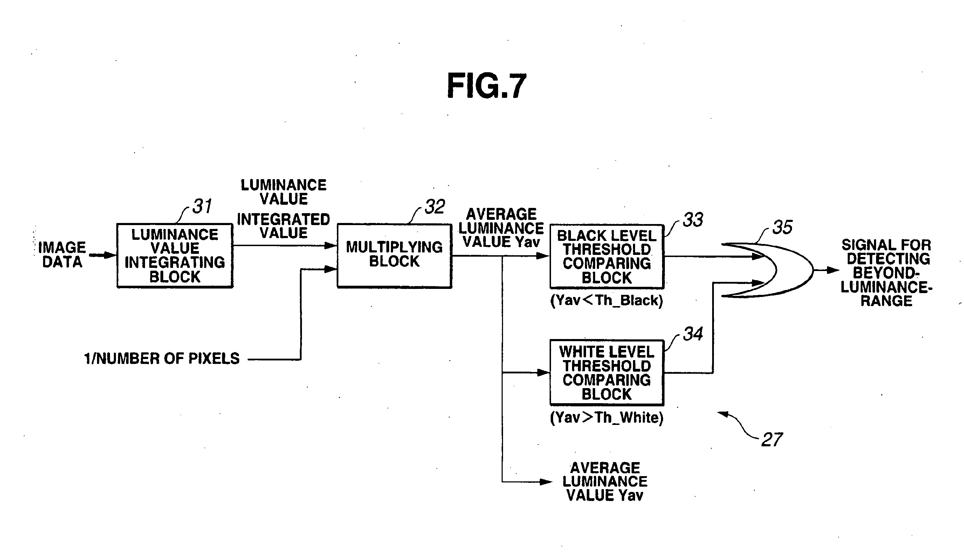

Fig. 7 shows the structure of the luminance range detecting block 27 shown in Fig. 6.

The luminance range detecting block 27 comprises: a luminance value integrating block 31 for integrating the luminance values of all the pixels of the inputted pixel signals; and a multiplying block 32 for calculating an average luminance value Yav of the image signal by dividing the integrated value of luminance values from the luminance value integrating block 31 by the number of pixels or multiplying it by 1/(number of pixels).

The luminance range detecting block 27 further comprises: a black level threshold value comparing block 33 for comparing whether or not the average luminance value Yav outputted from the multiplying block 32 is lower than a threshold value (Th_Black) of the black level; a white level threshold value comparing block 34 for comparing whether or not it is higher than a threshold value (Th_White) of the white level; and an OR circuit 35 into which output signals from the black level threshold value comparing block 33 and white level threshold value comparing block 34 are inputted.

The luminance range detecting block 27 outputs, from the OR circuit 35, a beyond-luminance-range signal indicating the determining result whether or not the image is extremely dark or extremely bright.

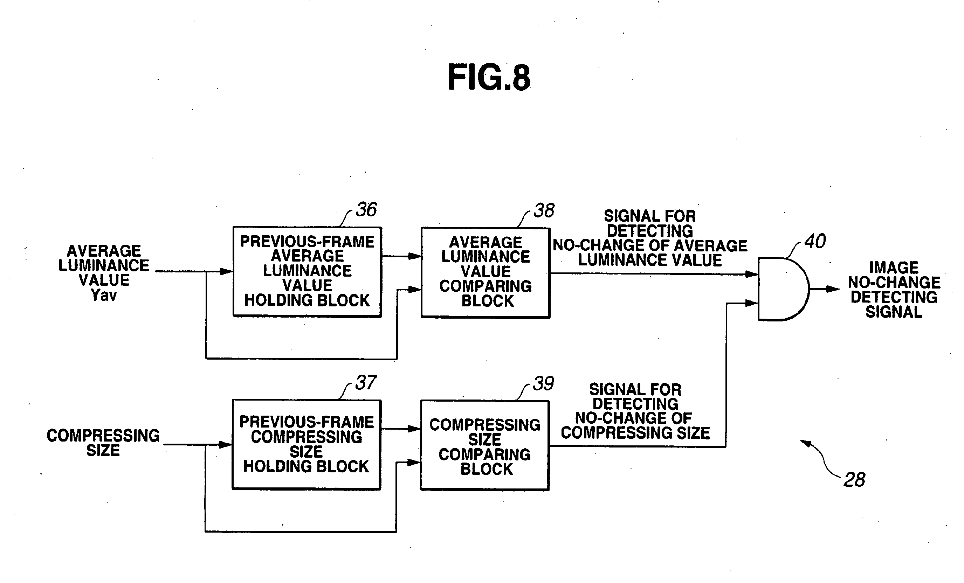

Fig. 8 shows an example of the image change detecting block 28 shown in Fig. 6. The image change detecting block 28 detects the image change based on the average luminance value Yav and the compressing size outputted from the compressing block 24.

Therefore, the average luminance value Yav and the compressing size are held in a previous-frame average luminance value holding block 36 and a previous-frame compressing size holding block 37 holding the average luminance value Yav and the compressing size of the frame before one frame.

The average luminance value Yav before one frame and the average luminance value Yav of the current frame are inputted to an average luminance value comparing block 38. The compressing size before one frame and the compressing size of the current frame are inputted to a compressing size comparing block 39.

The average luminance value comparing block 38 and compressing size comparing block 39 calculate the difference in average luminance values between the previous frame and the current frame and the difference in compressing sizes therebetween, respectively. If the absolutes of the difference are within a predetermined range, it is determined that the image does not change. Each non-change detecting signal of the average luminance value and compressing size is outputted to an AND circuit 40.

The AND circuit 40 outputs the image non-change detecting signal by the AND operation of the two non-change detecting signals of the average luminance value and compressing size.

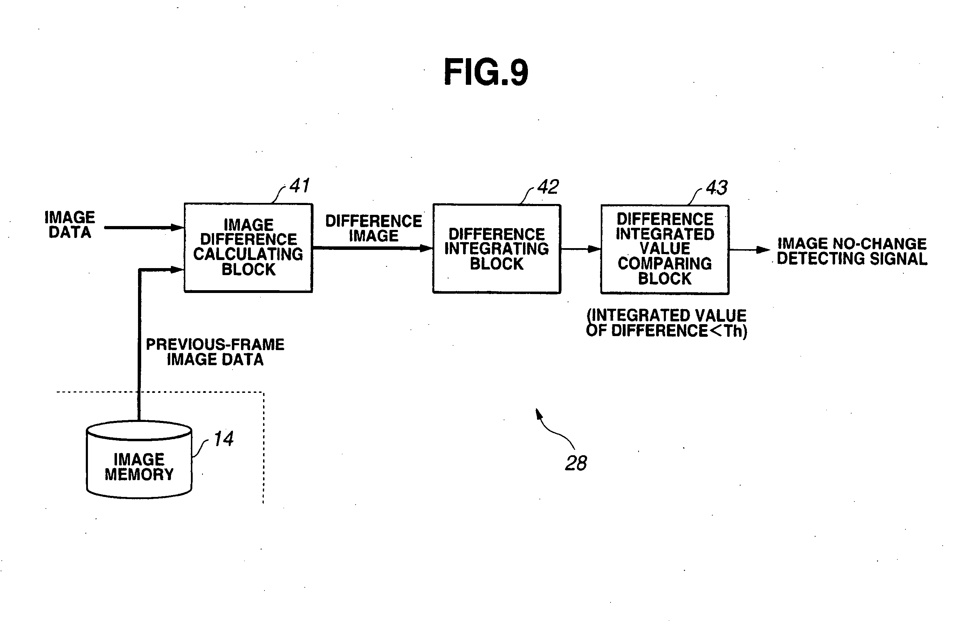

Fig. 9 shows the image change detecting block 28 according to a modification. According to the modification, the image change detecting block 28 reads the image of the current frame from the image memory 14 shown in Fig. 2, and the image of the previous frame and both the images are inputted to an image difference calculating block 41.

The image difference calculating block 41 calculates the difference between the current frame and the previous frame every pixel, and inputs the resultant difference image to a difference integrating block 42, thus to calculate the integrated value. The integrated value is inputted to a difference integrated value comparing block 43.

The difference integrated value comparing block 43 compares the integrated value of one frame with a predetermined threshold value (e.g., Th). If it is determined that the integrated value is lower than the threshold value, the difference integrated value comparing block 43 determines that the image does not change and outputs the image non-change detecting signal.

If it is determined that the image is not worth viewing, that is, the image is extremely bright or extremely dark, or the image is the same as that transmitted before, the difference integrated value comparing block 43 does not transmit the image data. Thus, the power consumption of the battery 21 is reduced.

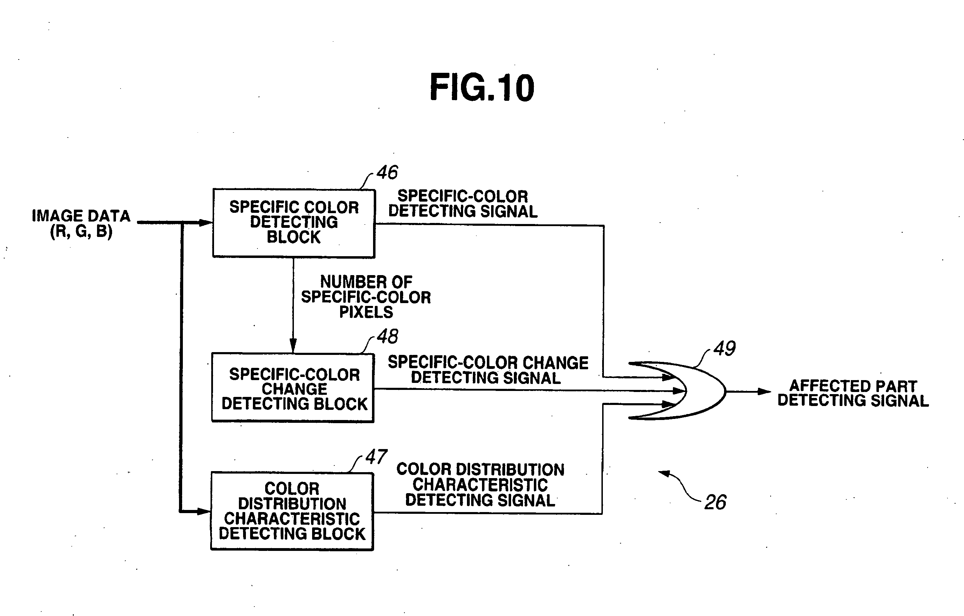

Fig. 10 shows the structure of the affected part detecting block 26 shown in Fig. 4.

According to the first embodiment, the affected part detecting block 26 detects the affected part which color-changes from the normal part (ulcer, tumor, hemorrhage, etc.), and comprises: a specific color detecting block 46 and a color distribution characteristic detecting block 47 for receiving image data (R, G, and B); a specific-color change detecting block 48 for detecting the change in specific color based on the number of pixels of the specific color from the specific color detecting block 46; and an OR circuit 49 for receiving the output signals from the three blocks 46 to 48.

The specific color detecting block 46 for receiving the image data (R, G, and B) detects the affected part by determining whether or not the affected part has a predetermined number of pixels in the stated or specific color space. In this case, the specific color detecting block 46 outputs a specific-color detecting signal to the OR circuit 49.

Further, the specific-color change detecting block 48 detects the affected part in the case that number of pixels in the stated color space peculiar to the affected part, namely number of pixels in the specific color has changed. In this case, the specific-color change detecting block 48 outputs a specific-color change detecting signal to the OR circuit 49.

The color distribution characteristic detecting block 47 calculates the hue and saturation of the inputted image data, detects the affected part based on the characteristics and outputs a color distribution characteristic detecting signal to the OR circuit 49 in this case. The change of specific color and the color distribution are detected in parallel therewith and thus the color-changed affected part having some individual difference can accurately be detected.

In the case shown in Fig. 10, upon detecting any of the specific color detecting signal, specific-color change detecting signal, and color distribution characteristic detecting signal, the affected part detecting signal is outputted to the control block 19 via the OR circuit 49.

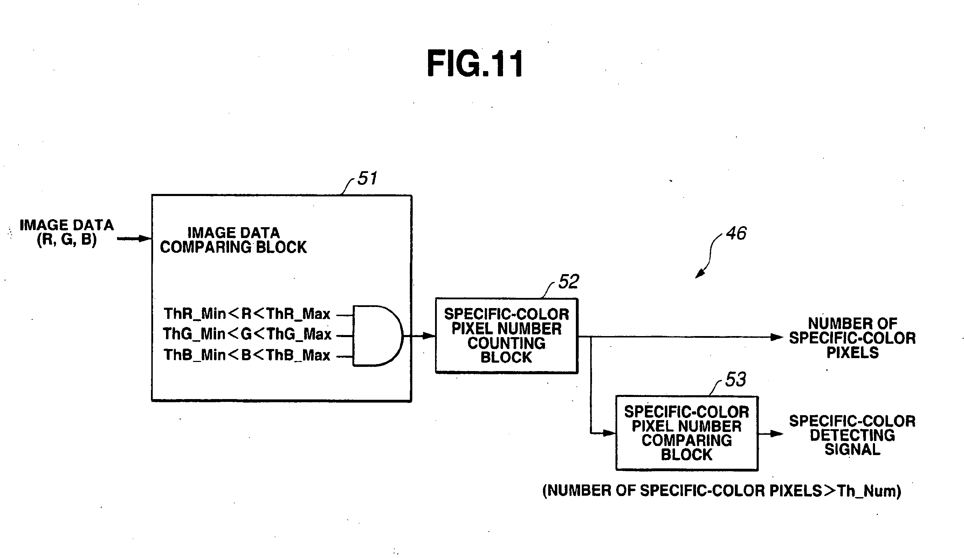

The structures and operations of the blocks shown in Fig. 10 will be described hereinbelow. Fig. 11 shows the structure of the specific color detecting block 46.

The specific color detecting block 46 compares the values of the image signals (R, G, and B shown in Fig. 10 according to the first embodiment) with the threshold value, that is, detects whether or not they are within a predetermined range by an image data comparing block 51.

Referring to Fig. 11, the image data comparing block 51 compares the values of the image signals (R, G, and B) with Th_Min < R < Th_Max, Th_Min < G < Th_Max, and Th_Min < B <Th_Max. Further, image data comparing block 51 outputs the resultant data to the AND circuit and obtains the result of the logical product from the AND circuit.

When all the image signals (R, G, and B) are within the predetermined range, the image data comparing block 51 outputs the resultant data as the specific-color pixel to a specific-color pixel number counting block 52 at the next stage. Further, the specific-color pixel number counting block 52 counts the number of pixels.

Thus, the specific-color pixel number counting block 52 detects the value (number of specific-color pixels) shared by the specific-color pixel in the image. The number of specific-color pixels is inputted to the specific-color change detecting block 48 shown in Fig. 10 and are inputted to a specific-color pixel number comparing block 53 shown in Fig. 11.

The specific-color pixel number comparing block 53 compares the number of specific-color pixels with a pre-determined threshold value Th_Num, and determines the detection of the specific color of the affected part if it is determined that the number of specific-color colors is Th_Num or more. The (value of) the number of specific-color pixels is outputted to the specific color detecting block 46.

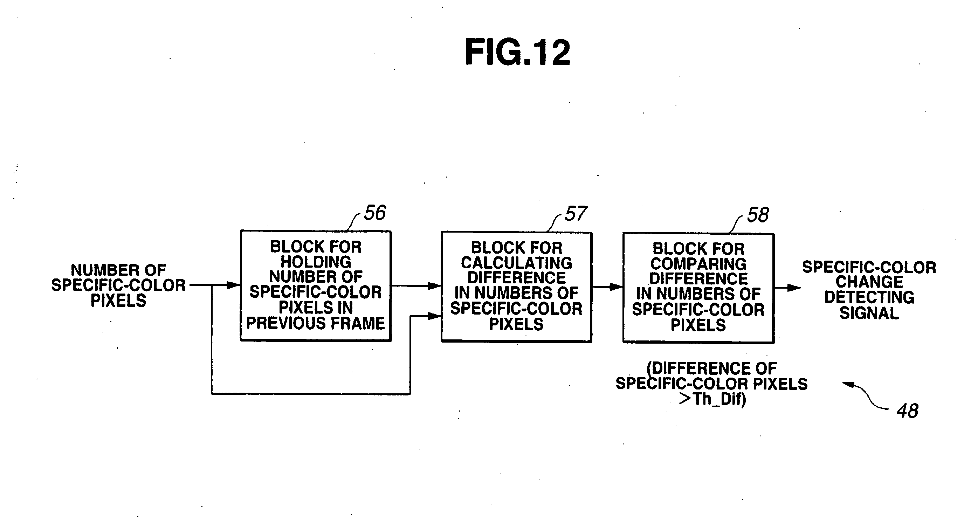

Fig. 12 shows the structure of the specific-color change detecting block 48 shown in Fig. 10.

A block 56 for holding the number of specific-color pixels in the previous frame and a block 57 for calculating the difference in number of specific-color pixels form the specific-color change detecting block 48. The number of specific-color pixels are inputted to the block 56 for holding the number of specific-color pixels in the previous frame and block 57 for calculating the difference in number of specific-color pixels from the specific color detecting block 46. The block 56 for holding the number of specific-color pixels in the previous frame holds the number of specific-color pixels of the previous frame.

The block 57 for calculating the difference in number of specific-color pixels calculates the difference in number of specific-color pixels between the previous frame and the current frame, and inputs the calculating result to a block 58 for comparing the difference in number of specific-color pixels. The block 58 for comparing the difference in number of specific-color pixels compares the difference with a threshold value Th_Dif, that is, determines whether or not the difference is a predetermined value or more. If it is determined that the difference is the threshold value Th_Dif or more, the change in specific color is detected and a specific-color change detecting signal is outputted.

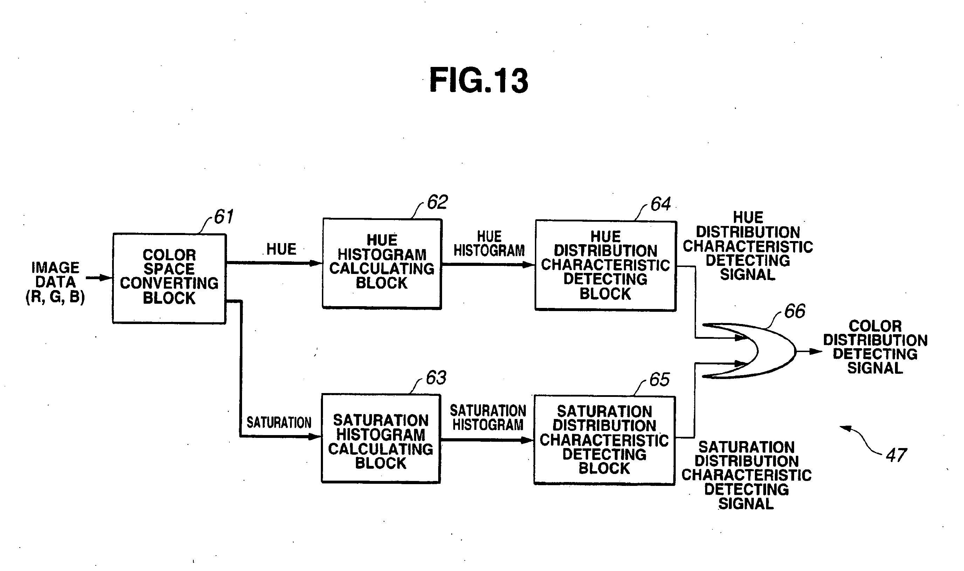

Fig. 13 shows the structure of the color distribution characteristic detecting block 47 shown in Fig. 10.

The color distribution characteristic detecting block 47 converts the inputted R-, G-, and B-images into the hue and saturation by a color space converting block 61. Next, a hue histogram calculating block 62 and a saturation histogram calculating block 63 detect the histograms (frequency distributions) of hue and saturation.

(Data of) the hue histogram and saturation histogram are inputted to a hue distribution characteristic detecting block 64 and a saturation distribution characteristic detecting block 65. The hue distribution characteristic detecting block 64 and saturation distribution characteristic detecting block 65 detect whether or not the histograms have predetermined characteristics, which will be described later. If it is determined that the histograms have the predetermined characteristics, the hue distribution characteristic detecting block 64 and saturation distribution characteristic detecting block 65 output, to an OR circuit 66, a signal for detecting the characteristic of the hue distribution and a signal for detecting the characteristic of the saturation distribution.

If it is detected that the histogram of any of the hue and saturation has the predetermined characteristic, a signal for detecting the characteristic of the color distribution is outputted via the OR circuit 66.

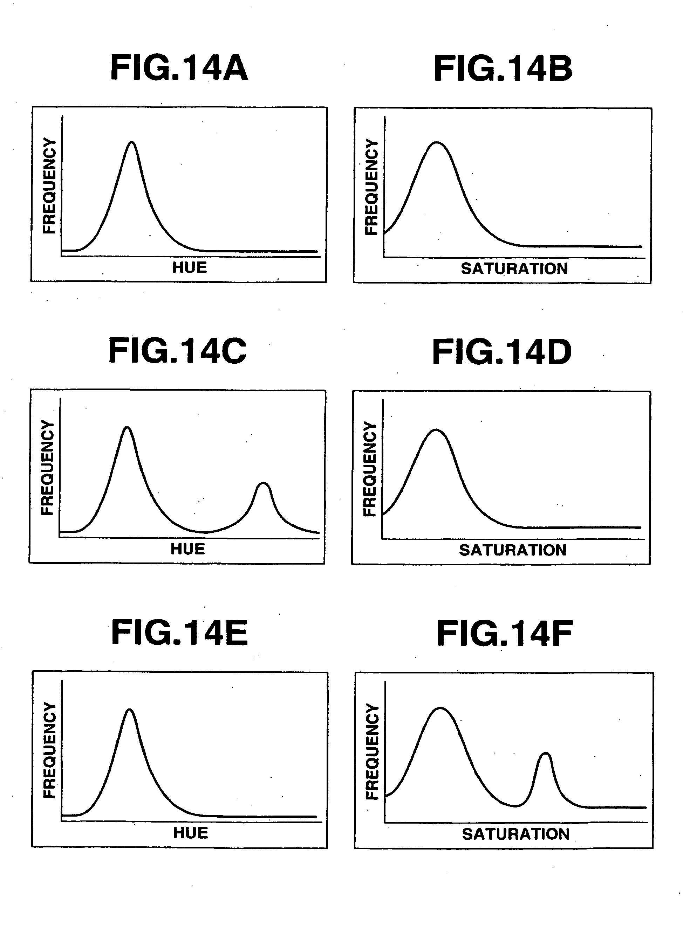

Fig. 14 shows examples of the characteristics of the color distribution of the hue and saturation of the affected part.

Figs. 14A and 14B show the example of characteristics of the hue and saturation at the normal part. Since the internal organ photographed at the normal part is uniform, the hue and saturation have the peak at one place.

Figs. 14C and 14D show examples of the characteristics of the hue and saturation in the case of photographing the color-changed part in which the changed color is generated by the ulcer or tumor. Since the hue at a part of the image is different, another peak is generated in addition to the peak of the normal part.

Figs. 14E and 14F show example of the characteristics of the hue and saturation in the case of photographing a part in which the color change is caused by the hemorrhage or the like. Although the hue is not changed in this case, the saturation at the normal part is different from that of the hemorrhage. Therefore, the saturation has another peak in addition to the peak which is generated at the normal part.

As mentioned above, when the image has the color-changed portion, the histograms of hue and saturation have a plurality of peaks at a predetermined distance.

Next, the detailed operation of blocks will be described.

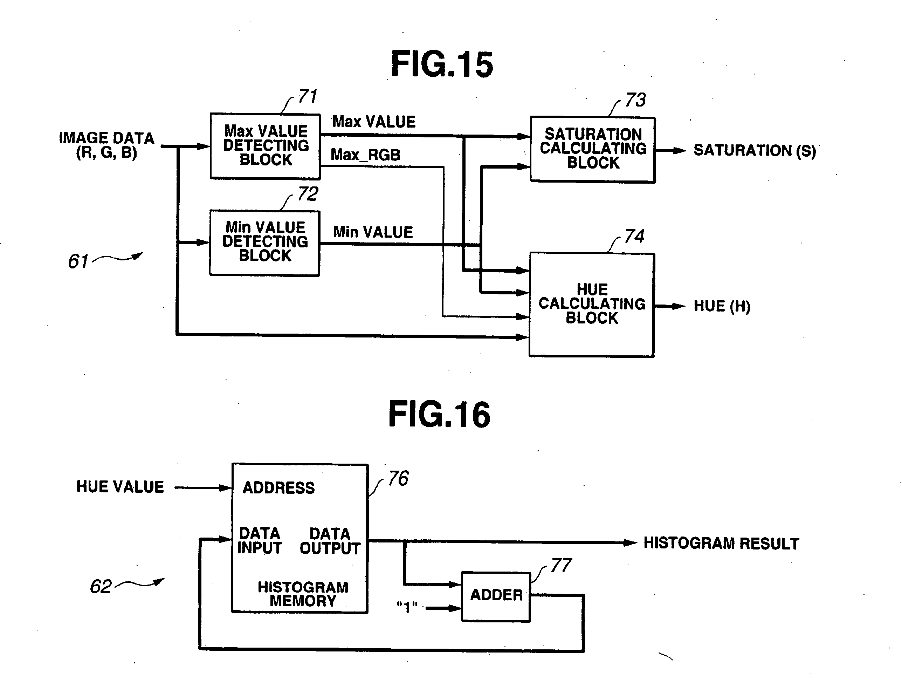

Fig. 15 shows one example of the color space converting block 61 shown in Fig. 13. The color space converting block 61 converts the image data inputted in the RGB space into the hue (H) and saturation (S).

Thus, the image data is inputted to a Max value detecting block 71 and a Min value detecting block 72. The Max value detecting block 71 and the Min value detecting block 72 compare the R-, G-, and B-values of the pixels in the inputted image data, select the maximum value and the minimum value, and output the selected values as a Max value and a Min value to a saturation calculating block 73 and a hue calculating block 74. The Max value detecting block 71 outputs, to the hue calculating block 74, a Max_RGB signal indicating that the Max value is any of R, G, and B. The image data is inputted to the hue calculating block 74.

The saturation calculating block 73 calculates the following saturation S.

Saturation S = (Max value - Min value)/(Max value) The saturation is calculated based on the above-mentioned Max value and Min value.

The hue calculating block 74 calculates the hue value by the following calculation based on a Max_RGB signal indicating the Max value is any of R, G, and B.

That is, when R has the Max value,

$$\text{Hue } H = (G - B)/(Max - Min).$$

Further, when G has the Max value,

$$\text{Hue } H = 2 + (B - R)/(Max - Min).$$

Furthermore, when B has the Max value,

$$\text{Hue } H = 4 + (R - G)/(Max - Min)$$

As mentioned above, the hues are calculated.

Fig. 16 shows the structure of the hue histogram calculating block 62 shown in Fig. 13. The hue histogram calculating block 62 comprises a histogram memory 76 and an adder 77 for adding one.

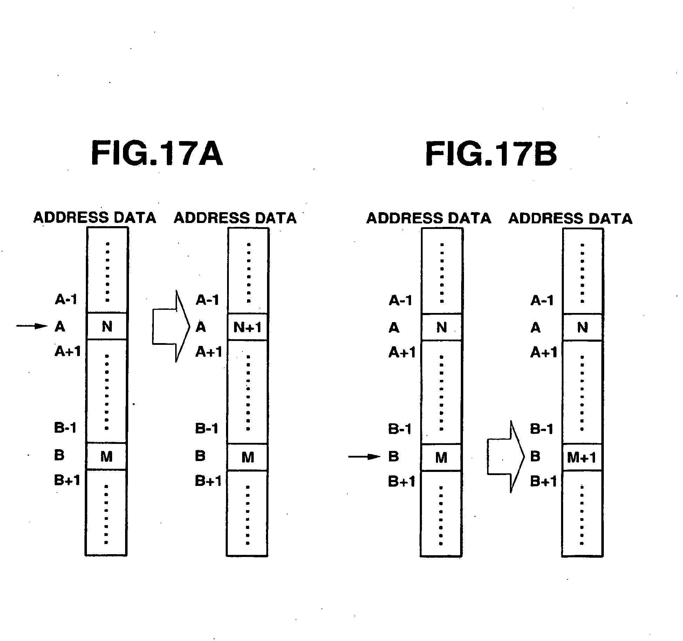

The hue value is inputted to the address of the histogram memory 76. After inputting the hue value, the value stored in the address is incremented by 1. Figs. 17A and 17B show the operation of the histogram memory 76.

Fig. 17A shows the case of inputting a hue value. Data N stored in the address A is incremented by 1 and data (N + 1) is stored. Fig. 17B shows the case of inputting a hue value. Data M stored in the address B is incremented by 1, and data (M + 1) is added. By repeating the above operation for all the pixels, the frequency distribution of the hue values is stored in the histogram memory 76.

The saturation histogram calculating block 63 shown in Fig. 13 has the above-mentioned structure. Thus, the structure and operation of the saturation histogram calculating block 63 are not described here.

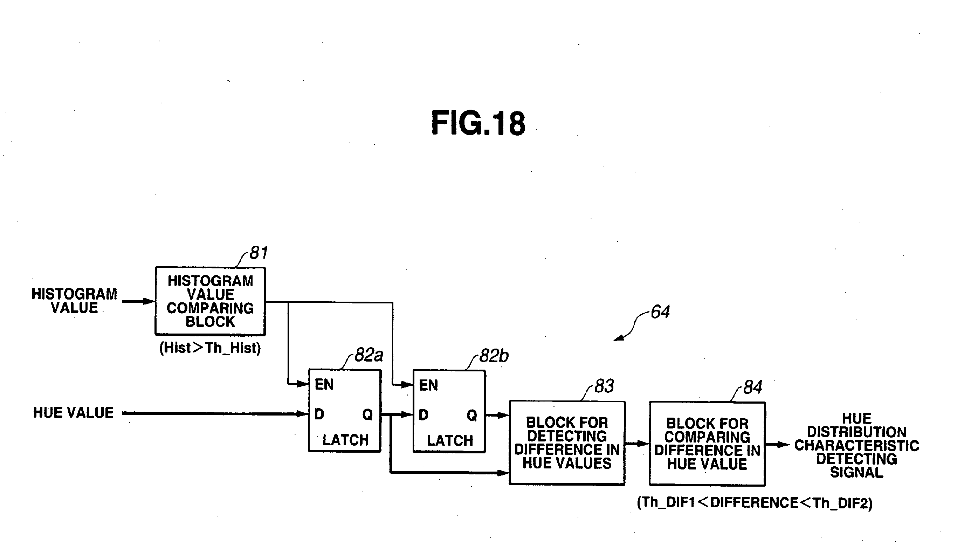

Fig. 18 shows the structure of hue distribution characteristic detecting block 64 in Fig. 13. The hue distribution characteristic detecting block 64 comprises: a histogram value comparing block 81 for comparing a histogram value Hist with a predetermined threshold value Th_Hist; latch circuits 82a and 82b for latching the corresponding hue values when the comparison result is the threshold value Th_Hist or more; and a block 83 for calculating the difference in hue values for calculating the difference between the latched hue values; and a block 84 for comparing the difference in hue values for comparing whether or not the difference is within a predetermined range.

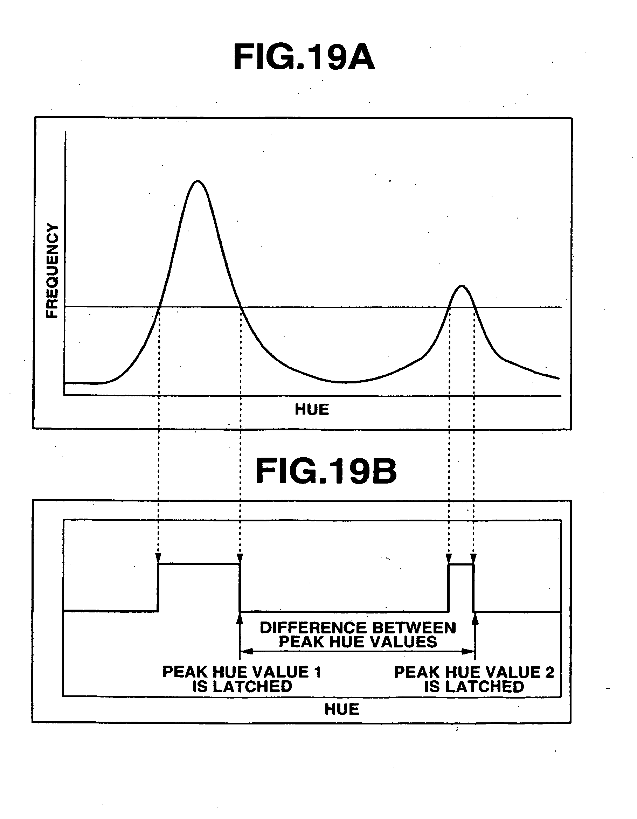

A description is given of the operation of the hue distribution characteristic detecting block 64 with reference to Figs. 19A and 19B.

Fig. 19A shows the histogram of the inputted hue values. Fig. 19B shows an explanatory diagram of the operation for comparing the histogram value of the hue value with the threshold value and for detecting the distance between the hue values.

As mentioned above, the histogram value comparing block 81 compares the histogram value of the hue value with the threshold value, thereby outputting the pulses near the position of the peak value of the histogram. The latch circuits 82a and 82b shown in Fig. 18 latch the corresponding hue values by the pulses. The block 83 for calculating the difference in hue values at the next stage calculates the distance between the hue values by using the latched hue values.

That is, as shown in Fig. 19B, the distance between the peak hue values is obtained. When the distance is within a predetermined range (referring to Fig. 18, Th_DIF1 < difference < Th_DIF2), the image has a portion with the hue different from that of the normal image and the block 84 for comparing the difference in hue values outputs a signal for detecting the characteristic of the hue distribution.

The saturation distribution characteristic detecting block 65 shown in Fig. 13 has the similar structure.

According to the first embodiment, the predetermined characteristic amount such as the number of pixels having the specific color in the image is detected based on the picked-up image. It is determined whether or not the detecting result is valid. Thus, the rate for transmitting the picked-up image to the extra-corporeal unit 4 is controlled and it is possible to set, to the proper state, the power consumption for transmitting the image with the large load by the battery 21.

Further, according to the first embodiment, it is possible to efficiently obtain the necessary image on the extra-corporeal unit 4 side. It is advantageous to eliminate or extremely reduce the troublesome operation for extracting the necessary image from the unneeded images according to the conventional art.

That is, it is possible to reduce the unnecessary power consumption for transmitting the image upon picking up the invalid image. The detailed image for diagnosis can be transmitted to the extra-corporeal unit 4 without suppressing the image transmitting rate upon picking up the valid image. Further, the electric energy of the battery 21 can effectively be used and the image for diagnosis can effectively be collected.

Even when the color of affected part is varied depending on the individual difference, the affected part such as changed color and hemorrhage is accurately detected without transmitting the unnecessary image. The detailed image for diagnosis is transmitted and the image diagnostic environment of the operator can be improved.

(Second embodiment)

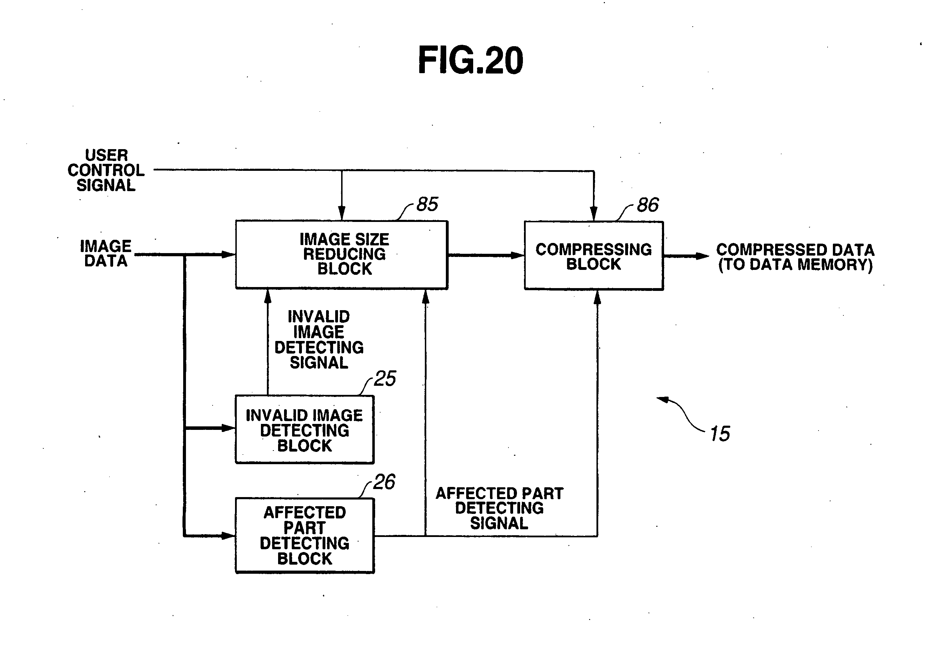

The second embodiment of the present invention will be described with reference to Figs. 20 to 25.

Fig. 20 shows the structure of the processing block 15 according to the second embodiment. The image data inputted to the processing block 15 is inputted to an image size reducing block 85, the invalid image detecting block 25, and the affected part detecting block 26.

The image size reducing block 85 controls the reduction of image size under the control of the invalid image detecting block 25 and the affected part detecting block 26.

That is, the image size reducing block 85 reduces the image size by the invalid image detecting signal from the invalid image detecting block 25 and suppresses the reduction of image size upon inputting the affected part detecting signal from the affected part detecting block 26.

The output image from the image size reducing block 85 is inputted to a compressing block 86. The compressing block 86 changes the compressing ratio by a control signal from the affected part detecting block 26 and outputs the compressed image to the communication block 17 shown in Fig. 2.

That is, the compressing block 86 reduces the compressing ratio of the image data and transmits, to the communication block 17, the compressed data which is compressed by the low compressing ratio upon inputting the affected part detecting signal from the affected part detecting block 26.

The communication block 17 transmits the compressed data to the extra-corporeal unit 4.

The invalid image detecting block 25 detects the invalid image (white compression, black compression, and non-change of obtained image). Further, the affected part detecting block 26 detects the absence or presence of the affected part or its similar part based on the image data.

The reducing ratio of image size and the compressing ratio are controlled by a user control signal outputted from the control block 19 based on a command received from the extra-corporeal unit 4. Further, The on/off operation of the control is performed by the invalid image signal and the affected part detecting signal. As mentioned above, the compressing ratio of the image size is controlled by inputting the command from the user.

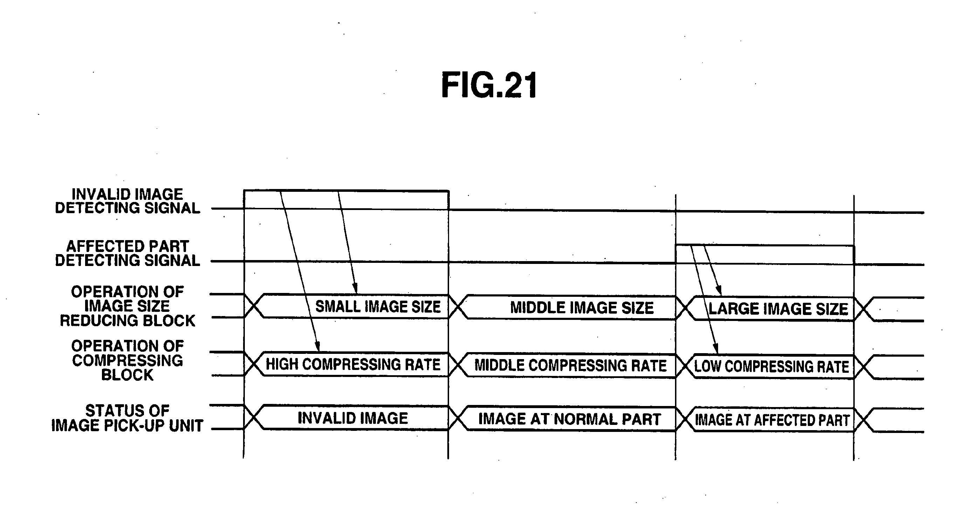

Fig. 21 shows a timing cart of the processing block 15.

Upon detecting the invalid image, the image size is minimized and the compressing ratio is maximized. Thus, the amount of transmitted data is suppressed to the minimum level. Because, mainly, information at the minimum level is transmitted to monitor the state of image pick-up unit 3.

Next, neither upon detecting the invalidity nor upon detecting the affected part, in other words, upon detecting the image at the normal part, the image size and the compressing ratio are set to the middle level. Because the image at the normal part for reference is transmitted to the extra-corporeal unit 4. When the affected part detection is active, the image size is not reduced because of increasing the amount of information of the affected-part image. Further, compressing ratio is reduced and the image is externally outputted with the highest quality.

The affected part detecting block 26 and the invalid image detecting block 25 shown in Fig. 20 have the same structures as those according to the first embodiment and therefore a description thereof is omitted.

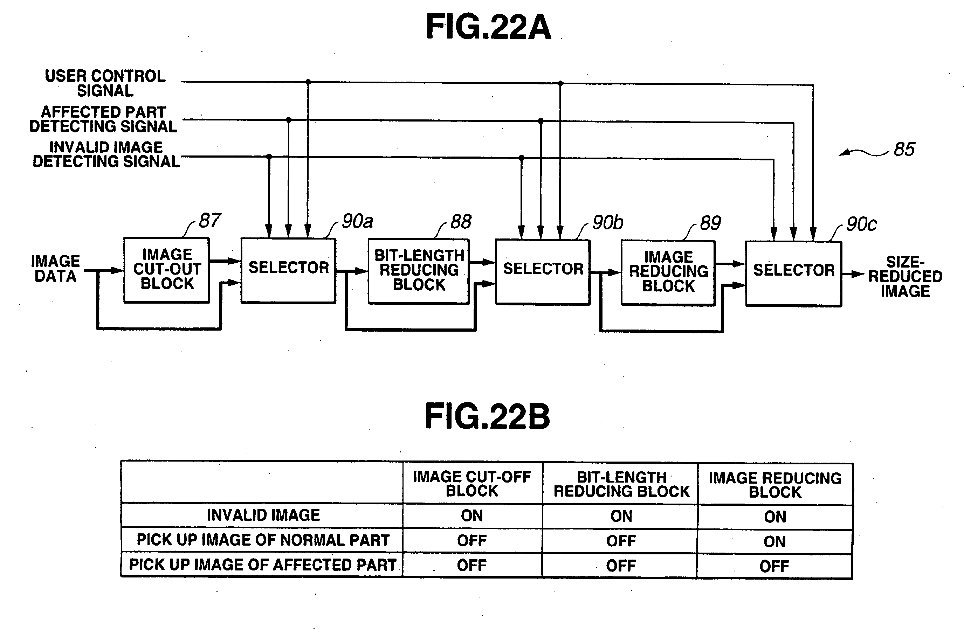

Fig. 22A shows the structure of the image size reducing block 85 according to the second embodiment.

The image size reducing block 85 comprises: an image cut-out block 87; a Bit-length reducing block 88; an image reducing block 89; and selectors 90a, 90b, and 90c for selecting the image from the blocks. The image size reducing block 85 controls the operation such as the size reduction from the original image based on the affected part detecting signal, invalid image signal, and user control signal.

Referring to Fig. 22B, as the determining result of the invalid image signal and affected part detecting signal, specifically, depending on the cases of the invalid image, picking up the normal part, and detecting the affected part, the image whose size is reduced from the original image is outputted via the selectors 90a to 90c.

For example, the image cut-out block 87 reduces the number of pixels by decreasing an angle of view (pixel size of the image) by cutting out the image.

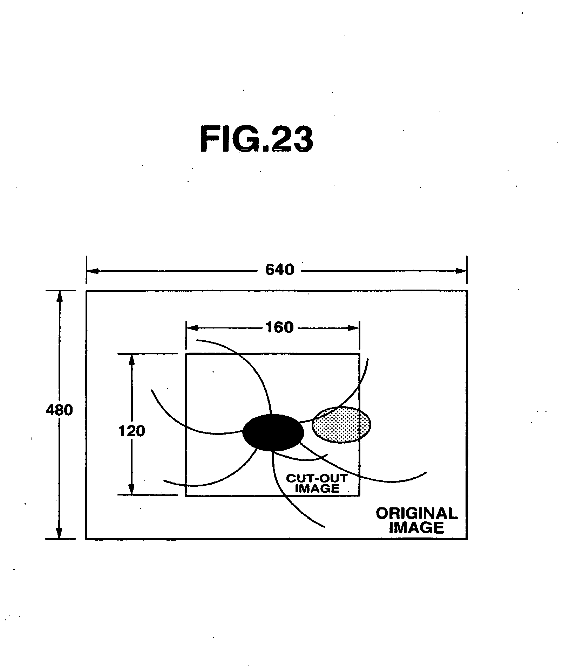

Fig. 23 shows an example of cutting out the image by the image cut-out block 87. In this example, only the center of the original image having (640 × 480) pixels is cut out. The center, specifically, the image having (160 × 120) pixels is outputted. Then, when the invalid image signal detects that the image is invalid, the cut-out image is outputted to the latter-stage side. When the invalid image signal does not detect that the image is invalid, the original image from which the image is not cut out is outputted to the latter-stage side.

The bit-length reducing block 88 shown in Fig. 22A reduces the image size by decreasing the bit length of the image.

According to the second embodiment, the gradation of 8 bits is reduced to that of 4 bits, thereby reducing the bit length of the image.

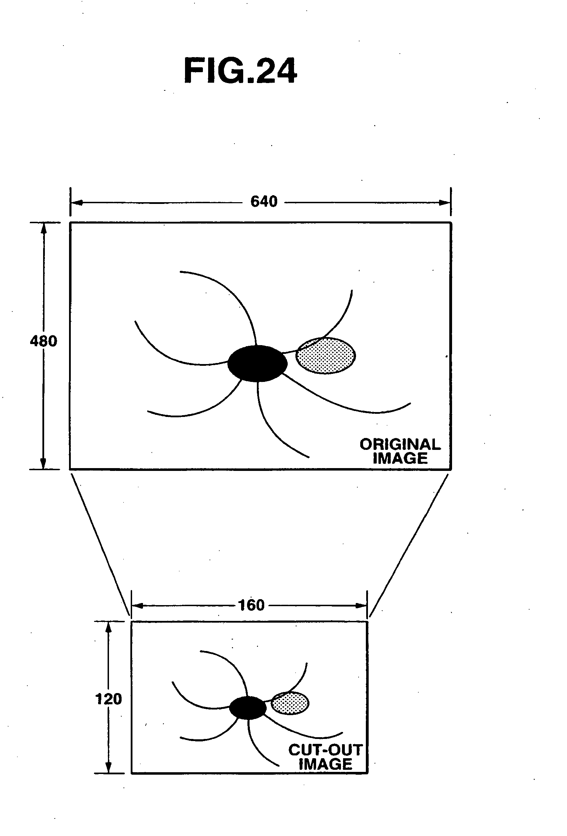

The image reducing block 89 thins out the pixels, and this example is shown in Fig. 24. In this example, the image having (640 × 480) pixels shown on the top side is reduced to the image having the (160 × 120) pixels by the thinning out the pixels as shown on the bottom side in Fig. 24. Since the simple thinning-out operation of pixels normally causes the problem on the image quality, the processing with the interpolation using the algorithm such as bi-linear and bi-cubic is performed.

According to the second embodiment, when it is determined that the image is invalid, the image is transmitted at the level for determining the state of the image pick-up unit 3, namely, the occurrence of white compression, black compression or stop. In this case, the image cut-out block cuts out the image of one part of the angle of view, and the Bit length is 4 bits by the Bit length reduction, thus to reduce the image.

Next, when neither the invalid image nor the affected part is detected, the image at the normal part is transmitted as the reference. Thus, the image cut-out operation and the reduction of Bit length stop and only the image is reduced, thus to output image.

Further, upon detecting the affected part, any image size is not reduced because of using the image for diagnosis with the highest image quality.

As mentioned above, it is possible to control the blocks by the user control signal which is transmitted by the command received from the extra-corporeal unit 4.

The compressing block 86 compresses the image data whose image size is reduced if necessary. According to the second embodiment, the image compression uses JPEG. In the case of the JPEG compression, the compressing ratio can arbitrarily be changed by a table of compressing parameters.

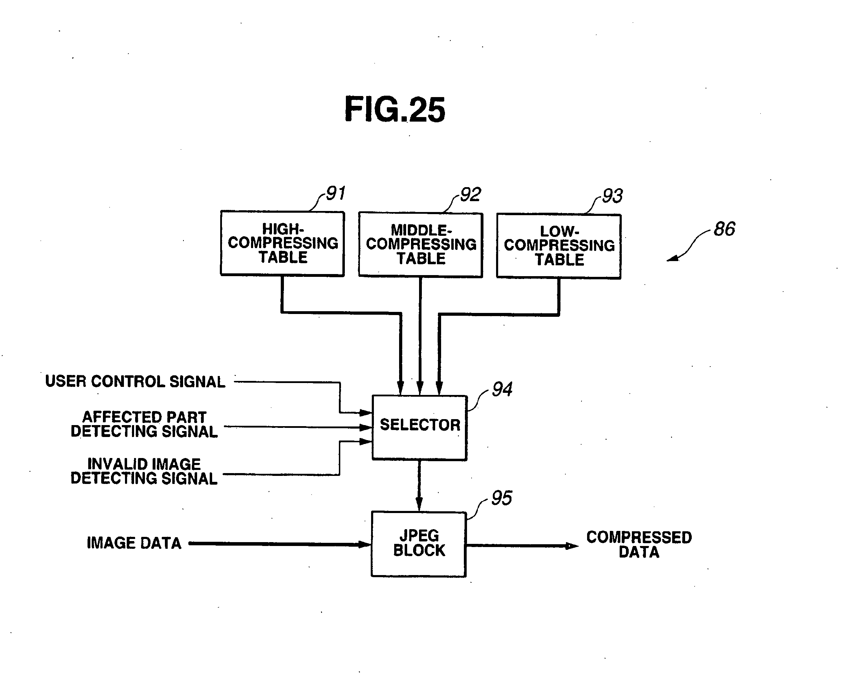

Fig. 25 shows the schematic structure of the compressing block 86 shown in Fig. 20. The compressing block 86 comprises: a high-compressing table 91, a middle-compressing table 92, and a low-compressing table 93 which compress the data by high, middle, and low compressing ratios, respectively; a selector 94 for selecting one of the high-, middle-, and low-compressing tables 91 to 93; and a JPEG block 95 for JPEG-compressing the data by the selecting compressing table.

Upon inputting the invalid image detecting signal, the compressing table is switched to that with the high compressing ratio. Upon inputting the affected part detecting signal, the compressing table is switched to that with the low compressing ratio. Upon detecting neither the invalid image detecting signal nor the affected part detecting signal, the compressing table with middle compressing ratio is used.

Similarly to the image size reducing block, it is possible to control the tables by the user control signal which is transmitted by the command received from the extra-corporeal unit 4.

As mentioned above, according to the second embodiment, the image size and the compressed data size are switched depending on the determining result of the image importance (validity) and the compression and communication time is reduced. The consumption power is reduced and the image with high quality necessary for diagnosis can be transmitted.

The above control suppresses the power consumption of the battery 21 in the case of the unnecessary image and further enables the long use.

The present invention can variously be modified. According to the first and second embodiments, the transmitting interval of the image data and the size of transmitted data are independently controlled. However, the combination of the above two control operations can be used.

Further, other applications are considered, e.g., it is detected whether or not the area having the desired color has a predetermined size upon detecting the affected part and it is used by combining the above two control methods. These applications belong to the present invention.

As mentioned above, according to the present invention, it is possible to control the amount of images transmitted to the extra-corporeal unit to the proper value without arranging the sensor in the image pick-up unit.

Industrial Applicability

As described above, the endoscope image pick-up apparatus of the present invention picks up the image in the body by the image pick-up unit inserted in the body and transmits the image by radio to the extra-corporeal unit which is arranged outside the body. The amount of images transmitted to the extra-corporeal unit side is properly controlled, thereby obtaining the image suitable to the endoscopic examination.

**Claims**

1. An endoscope image pick-up apparatus (1) including an image pick-up unit (3) for picking up an image of the body and for transmitting the image by radio to an extra-corporeal unit (4), the image pick-up unit (3) comprising:

    image pick-up means (13) for capturing the image;
    data processing means (15) for performing the processing for reducing the data amount of the image obtained by the image pick-up means (13) at a plurality of ratios;
    data transmitting means (17, 19) for transmitting the data processed by the data processing means (15) to the extra-corporeal unit (4);
    characteristic amount detecting means (25, 26) for detecting a predetermined amount of characteristics based on the image; and
    determining means (25, 26) for determining based on the amount of characteristics whether or not the image is valid
    wherein the data processing means (15) is adapted to control a reducing ratio of data amount in accordance with the determining result outputted by the determining means (25, 26);
    wherein the characteristic amount detecting means (25, 26) include histogram determining means (47, 61-66) for detecting the characteristic amount, hue histogram and saturation histogram of the image from the image data and
    wherein the histogram determining means (47, 61-66) outputs a color distribution detecting signal indicating that the image is valid, when the hue histogram shows that a distance between two peak hue values at a threshold value or more is within a predetermined range, or when the saturation histogram shows that a distance between two peak saturation values at a threshold value or more is within a predetermined range.

2. An endoscope image pick-up apparatus (1) according to Claim 1, wherein the determining means (25, 26) comprises:

    invalidity determining means (25) for determining whether the image is valid or invalid; and
    target image determining means (26) for determining whether or not the image is a target image,
    wherein, based on the combination of the determining results at a plurality of stages, the image is determined to be valid and the determining result is outputted.

3. An endoscope image pick-up apparatus (1) according to Claim 1, wherein the characteristic amount detecting means (26) comprises pixel number detecting means (52) for detecting the number of pixels having a specific color in the image as the characteristic amount, and
    the determining means (26) determines that the image is valid when the number of specific-color pixels is a predetermined threshold value or more.

4. An endoscope image pick-up apparatus (1) according to Claim 1, wherein the characteristic amount detecting means (26) comprises:

pixel number detecting means (52) for detecting the number of pixels having a specific color in the image;

pixel number storing means (56) for storing the detected number of pixels; and

pixel number comparing and calculating means (58) for comparing and calculating the past number of pixels stored in the pixel number storing means (56) and the current number of pixels and for outputting the change amount of number of pixels as the characteristic amount,

wherein the determining means (26) is adapted to determine that the image is valid when the change amount of the number of pixels is a predetermined threshold value or more.

5. An endoscope image pick-up apparatus (1) according to Claim 1, wherein the characteristic amount detecting means (26) comprises:

color distribution comparing and calculating means (47, 48) for comparing and calculating the detected hue histogram and saturation histogram and a predetermined hue histogram and saturation histogram and for outputting an error of the hue histogram and saturation histogram as the characteristic amount, and

the determining means (26) is adapted to determine that the image is valid when the error of the hue histogram and saturation histogram is a predetermined threshold value or less.

6. An endoscope image pick-up apparatus (1) according to Claim 1, wherein the characteristic amount detecting means (25) comprises luminance average value calculating means (32) for detecting an average value of luminance values in the image as the characteristic amount,

wherein the determining means (26) is adapted to determine that the image is valid when the average value of luminance is within a predetermined range.

7. An endoscope image pick-up apparatus (1) according to Claim 1, wherein the characteristic amount detecting means (25) comprises:

luminance average value calculating means (32) for detecting an average value of luminance values in the image;

luminance average value storing means (36) for storing the luminance average value; and

luminance average value comparing and calculating means(38) for comparing and calculating the past luminance average value stored in the luminance average value storing means (36) and the current luminance average value and for outputting the change amount of the luminance average value as the characteristic amount,

wherein the determining means (26) is adapted to determine that the image is valid when the change amount of the luminance average value is a predetermined threshold value or more.

8. An endoscope image pick-up apparatus (1) according to Claim 1, wherein the characteristic amount detecting means (25) comprises:

image storing means (14) for storing the picked-up image; and

image data difference calculating means (41) for calculating the difference between the past image data stored in the image storing means (14) and the current image data and for outputting the calculated difference as the characteristic amount,

wherein the determining means (43) is adapted to determine that the image is valid when the difference is a predetermined value or more.

9. An endoscope image pick-up apparatus (1) according to Claim 1, wherein the data processing means (15) comprises image reducing means (85) for reducing image data at a plurality of reducing ratios.

10. An endoscope image pick-up apparatus (1) according to Claim 1, wherein the data processing means (15) comprises bit-length adjusting means (88) for switching the bit length of the image data to a plurality of lengths.

11. An endoscope image pick-up apparatus (1) according to Claim 1, wherein the data processing means (15) comprises image cut-out means (87) for cutting out a part of the image data and for outputting the cut-out image.

12. An endoscope image pick-up apparatus (1) according to Claim 1, wherein the data processing means (15) comprises compressing means (86) for compressing the image data at a plurality of compressing ratios.

13. An endoscope image pick-up apparatus (1) according to Claim 1, wherein the image pick-up unit (3) comprises command receiving means (17, 19) for receiving a plurality of types of commands from the extra-corporeal unit (4), and

the data processing means (15) is adapted to control a reducing ratio of data amount based on the command received by the command receiving means (17, 19), and to invalidate the control of the reducing ratio of data amount by the determining means (25, 26) based on another command received by the command receiving means (17, 19).

**14.** An endoscope image pick-up apparatus (1) according to Claim1,
wherein the data processing means (15) comprises
compressing means (24) for compressing the image obtained by the image pick-up means (13);
wherein the data transmitting means (17, 19) is adapted for transmitting the data compressed by the compressing means (24) to the extra-corporeal unit (7) at a plurality of transmitting rates; and
wherein the determining means (25, 26) is further adapted for determining the validity of the image by comparing the data size compressed by the compressing means (24) with a predetermined threshold value, and
wherein the data transmitting means (17, 19) controls the data transmitting rate in accordance with the determining result outputted by the determining means (25, 26).

**15.** An endoscope image pick-up apparatus (1) according to Claim 14, wherein the image pick-up unit (3) comprises command receiving means (17, 19) for receiving a plurality of types of commands from the extra-corporeal unit (4), and the data transmitting means (17) is adapted to control a data transmitting rate based on the command received by the command receiving means (17, 19), and invalidates the control of the data transmitting rate by the determining means (25, 26) based on another command received by the command receiving means (17, 19).

**16.** An endoscope image pick-up apparatus (1) according to Claim 1,
wherein the data processing means (15) comprises compressing means (24) for compressing the image obtained by the image pick-up means (13);
wherein the data transmitting means (17) is adapted for transmitting the data compressed by the compressing means (24) to the extra-corporeal unit (4) at a plurality of transmitting rates;
wherein the image pick-up unit further comprises:

compressed data size storing means (37) for storing the data size compressed by the compressing means (24);
compressed data size difference calculating means (39) for calculating the difference between the past compressed data size stored in the compressed data size storing means (39) and the current compressed data size; and
wherein the determining means (17) is adapted for comparing the difference in the compressed data size with a predetermined threshold value and for determining whether or not the image is valid, and
wherein the data transmitting means (17) is adapted to control a data transmitting rate in accordance with the determining result outputted by the determining means (39).

**17.** An endoscope image pick-up apparatus according to Claim 16, wherein the image pick-up unit (3) comprises command receiving means (17, 19) for receiving a plurality of types of commands from the extra-corporeal unit (4), and the data transmitting means (17) controls a data transmitting rate based on the command received by the command receiving means (17, 19), and invalidates the control of the data transmitting rate by the determining means (39) based on another command received by the command receiving means (17, 19).

**18.** An endoscope image pick-up apparatus (1) according to Claim 1, wherein the image pick-up unit (3) further comprises:

storing means (4) for storing the image obtained by the image pick-up means (3);
wherein the processing means (15) is adapted for reading the image from the storing means (14) and for performing predetermined processing,
wherein, upon storing the image, the storing means (14) is adapted to operate at the high-speed clock similar to that of the image pick-up means (3) and, upon processing the image, the storing means (14) operates at the low-speed clock similar to that of the processing means (15).

**Patentansprüche**

**1.** Endoskopbildaufnahmegerät (1), das eine Bildaufnahmeeinheit (3) zum Aufnehmen eines Bilds des Körpers und zum Übertragen des Bilds über Funk an eine sich außerhalb des Körpers befindende Einheit (4) umfasst, wobei die Bildaufnahmeeinheit (3) umfasst:

eine Bildaufnahmeeinrichtung (13) zum Aufnehmen des Bilds;

eine Datenverarbeitungseinrichtung (15) zum Durchführen der Verarbeitung zum Reduzieren der Datenmenge des durch die Bildaufnahmeeinrichtung (13) erhaltenen Bilds bei einer Mehrzahl von Verhältnissen;

eine Datenübertragungseinrichtung (17, 19) zum Übertragen der durch die Datenverarbeitungseinrichtung (15) verarbeiteten Daten zu der sich außerhalb des Körpers befindenden Einheit (4);

eine Einrichtung (25, 26) zur Erfassung einer charakteristischen Menge zum Erfassen einer vorbestimmten Menge von Charakteristiken basierend auf dem Bild;

eine Bestimmungseinrichtung (25, 26) zum Bestimmen, ob das Bild gültig ist oder nicht basierend auf der Menge von Charakteristiken,

wobei die Datenverarbeitungseinrichtung (15) dazu eingerichtet ist, ein Reduzierungsverhältnis einer Datenmenge gemäß dem durch die Bestimmungseinrichtung (25, 26) ausgegebenen Bestimmungsergebnis zu steuern;

wobei die Einrichtung (25, 26) zur Erfassung einer charakteristischen Menge eine Histogrammbestimmungseinrichtung (47, 61-66) zum Erfassen der charakteristischen Menge, eines Hue-Histogramms und eines Sättigungshistogramms des Bilds aus den Bilddaten umfasst, und

wobei die Histogrammbestimmungseinrichtung (47, 61-66) ein Farbverteilungserfassungssignal ausgibt, das anzeigt, dass das Bild gültig ist, wenn das Hue-Histogramm zeigt, dass ein Abstand zwischen zwei Spitzen-Huewerten bei einem Schwellwert oder mehr innerhalb eines vorbestimmten Bereichs liegt, oder wenn das Sättigungshistogramm zeigt, dass ein Abstand zwischen zwei Spitzen-Sättigungswerten bei einem Schwellwert oder mehr innerhalb eines vorbestimmten Bereichs liegt.

2. Endoskopbildaufnahmegerät (1) gemäß Anspruch 1, wobei die Bestimmungseinrichtung (25, 26) umfasst:

eine Ungültigkeitsbestimmungseinrichtung (25), zum Bestimmen, ob das Bild gültig oder ungültig ist; und

eine Zielbildbestimmungseinrichtung (26) zum Bestimmen, ob das Bild ein Zielbild ist oder nicht,

wobei, basierend auf der Kombination der Bestimmungsergebnisse bei einer Mehrzahl von Stufen, bestimmt wird, dass das Bild gültig ist und das Bestimmungsergebnis ausgegeben wird.

3. Endoskopbildaufnahmegerät (1) gemäß Anspruch 1, wobei die Einrichtung (26) zur Erfassung einer charakteristischen Menge eine Pixelzahlerfassungseinrichtung (52) zum Erfassen der Anzahl von Pixeln mit einer spezifischen Farbe in dem Bild als die charakteristischen Menge umfasst und

die Bestimmungseinrichtung (26) bestimmt, dass das Bild gültig ist, wenn die Anzahl von Pixeln mit einer spezifischen Farbe ein vorbestimmter Schwellwert oder mehr ist.

4. Endoskopbildaufnahmegerät (1) gemäß Anspruch 1, wobei die Einrichtung (26) zur Erfassung einer charakteristischen Menge umfasst:

eine Pixelzahlerfassungseinrichtung (52) zum Erfassen der Anzahl von Pixeln mit einer spezifischen Farbe in dem Bild;

eine Pixelzahlspeichereinrichtung (56) zum Speichern der erfassten Anzahl von Pixeln; und

eine Pixelzahlvergleichs- und Berechnungseinrichtung (58) zum Vergleichen und Berechnen der vorhergehenden Anzahl von Pixeln, die in der Pixelzahlspeichereinrichtung (56) gespeichert ist, und der gegenwärtigen Anzahl von Pixeln und zum Ausgeben des Änderungsbetrags der Anzahl von Pixeln als die charakteristische Menge,

wobei die Bestimmungseinrichtung (26) dazu eingerichtet ist, zu bestimmen, dass das Bild gültig ist, wenn der Änderungsbetrag der Anzahl von Pixeln ein vorbestimmter Schwellwert oder mehr ist.

5. Endoskopbildaufnahmegerät (1) gemäß Anspruch 1, wobei die Einrichtung (26) zur Erfassung einer charakteristischen Menge umfasst:

eine Farbverteilungsvergleichs - und Berechnungseinrichtung (47, 48) zum Vergleichen und Berechnen des erfassten Hue-Histogramms und des Sättigungshistogramms und eines vorbestimmten Hue-Histogramms und eines Sättigungshistogramms und zum Ausgeben eines Fehlers des Hue-Histogramms und des Sättigungshistogramms als die charakteristischen Menge, und

die Bestimmungseinrichtung (26) dazu eingerichtet ist, zu bestimmen, dass das Bild gültig ist, wenn der Fehler des Hue-Histogramms und des Sättigungshistogramms ein vorbestimmter Schwellwert oder mehr ist.

6. Endoskopbildaufnahmegerät (1) gemäß Anspruch 1, wobei die Einrichtung (26) zur Erfassung einer charakteristischen Menge eine Helligkeitsdurchschnittswertberechnungseinrichtung (32) zum Erfassen eines Durchschnittswerts von Helligkeitswerten in dem Bild als die charakteristischen Menge umfasst,
wobei die Bestimmungseinrichtung (26) dazu eingerichtet ist, zu bestimmen, dass das Bild gültig ist, wenn der Durchschnittswert der Helligkeit innerhalb eines vorbestimmten Bereichs liegt.

7. Endoskopbildaufnahmegerät (1) gemäß Anspruch 1, wobei die Einrichtung (25) zur Erfassung einer charakteristischen Menge umfasst:

   eine Helligkeitsdurchschnittswertberechnungseinrichtung (32) zum Erfassen eines Durchschnittswerts von Helligkeitswerten in dem Bild;
   eine Helligkeitsdurchschnittswertspeichereinrichtung (36) zum Speichern des Helligkeitsdurchschnittswerts; und
   eine Helligkeitsdurchschnittswertvergleichs- und Berechnungseinrichtung (38) zum Vergleichen und Berechnen des vorhergehenden Helligkeitsdurchschnittswerts, der in der Helligkeitsdurchschnittswertspeichereinrichtung (36) gespeichert ist, und des gegenwärtigen Helligkeitsdurchschnittswerts und zum Ausgeben des Änderungsbetrags des Helligkeitsdurchschnittswerts als die charakteristische Menge,
   wobei die Bestimmungseinrichtung (26) dazu eingerichtet ist, zu bestimmen, dass das Bild gültig ist, wenn der Änderungsbetrag des Helligkeitsdurchschnittswerts ein vorbestimmter Schwellwert oder mehr ist.

8. Endoskopbildaufnahmegerät (1) gemäß Anspruch 1, wobei die Einrichtung (25) zur Erfassung einer charakteristischen Menge umfasst:

   eine Bildspeichereinrichtung (14) zum Speichern des aufgenommenen Bilds; und
   eine Bilddatendifferenzberechnungseinrichtung (41) zum Berechnen der Differenz zwischen den vorhergehenden Bilddaten, die in der Bildspeichereinrichtung (14) gespeichert sind, und den gegenwärtigen Bilddaten und zum Ausgeben der berechneten Differenz als die charakteristische Menge,
   wobei die Bestimmungseinrichtung (43) dazu eingerichtet ist, zu bestimmen, dass das Bild gültig ist, wenn die Differenz ein vorbestimmter Schwellwert oder mehr ist.

9. Endoskopbildaufnahmegerät (1) gemäß Anspruch 1, wobei die Datenverarbeitungseinrichtung (15) eine Bildreduzierungseinrichtung (85) zum Reduzieren von Bilddaten bei einer Mehrzahl von Reduzierungsverhältnissen umfasst.

10. Endoskopbildaufnahmegerät (1) gemäß Anspruch 1, wobei die Datenverarbeitungseinrichtung (15) eine Bitlängenanpassungseinrichtung (88) zum Umschalten der Bitlänge der Bilddaten in eine Mehrzahl von Längen umfasst.

11. Endoskopbildaufnahmegerät (1) gemäß Anspruch 1, wobei die Datenverarbeitungseinrichtung (15) eine Bildausschneideeinrichtung (87) zum Ausschneiden eines Teils der Bilddaten und zum Ausgeben des ausgeschnittenen Bilds umfasst.

12. Endoskopbildaufnahmegerät (1) gemäß Anspruch 1, wobei die Datenverarbeitungseinrichtung (15) eine Komprimierungseinrichtung (86) zum Komprimieren der Bilddaten bei einer Mehrzahl von Komprimierungsverhältnissen umfasst.

13. Endoskopbildaufnahmegerät (1) gemäß Anspruch 1, wobei die Bildaufnahmeeinheit (3) eine Befehlsempfangseinrichtung (17, 19) zum Empfangen einer Mehrzahl von Befehlsarten von der sich außerhalb des Körpers befindenden Einheit (4) umfasst und
die Datenverarbeitungseinrichtung (15) dazu eingerichtet ist, ein Reduzierungsverhältnis einer Datenmenge basierend auf dem durch die Befehlsempfangseinrichtung (17, 19) empfangenen Befehl zu steuern und die Steuerung des Reduzierungsverhältnisses einer Datenmenge durch die Bestimmungseinrichtung (25, 26) basierend auf einem durch die Befehlsempfangseinrichtung (17, 19) empfangenen anderen Befehl außer Kraft zu setzen.

14. Endoskopbildaufnahmegerät (1) gemäß Anspruch 1, wobei die Datenverarbeitungseinrichtung (15)
eine Komprimierungseinrichtung (24) zum Komprimieren des durch die Bildaufnahmeeinrichtung (13) erhaltenen Bilds umfasst;
wobei die Datenübertragungsseinsrichtung (17, 19) dazu eingerichtet ist, die durch die Komprimierungseinrichtung (24) komprimierten Daten bei einer Mehrzahl von Übertragungsraten an die sich außerhalb des Körpers befindende Einheit (7) zu übertragen; und
wobei die Bestimmungseinrichtung (25, 26) ferner dazu eingerichtet ist, die Gültigkeit des Bilds durch Vergleichen der durch die Komprimierungseinrichtung (24) komprimierten Datengröße mit einem vorbestimmten Schwellwert zu bestimmen, und

wobei die Datenübertragungseinrichtung (17, 19) die Datenübertragungsrate gemäß dem durch die Bestimmungseinrichtung (25, 26) ausgegebenen Bestimmungsergebnis steuert.

15. Endoskopbildaufnahmegerät (1) gemäß Anspruch 14, wobei die Bildaufnahmeeinheit (3) eine Befehlsempfangseinrichtung (17, 19) zum Empfangen einer Mehrzahl von Befehlsarten von der sich außerhalb des Körpers befindenden Einheit (4) umfasst und

die Datenübertragungseinrichtung (17) dazu eingerichtet ist, eine Datenübertragungsrate basierend auf dem durch die Befehlsempfangseinrichtung (17, 19) empfangenen Befehl zu steuern und die Steuerung der Datenübertragungsrate durch die Bestimmungseinrichtung (25, 26) basierend auf einem durch die Befehlsempfangseinrichtung (17, 19) empfangenen anderen Befehl außer Kraft zu setzen.

16. Endoskopbildaufnahmegerät (1) gemäß Anspruch 1,

wobei die Datenverarbeitungseinrichtung (15) eine Komprimierungseinrichtung (24) zum Komprimieren des durch die Bildaufnahmeeinrichtung (13) erhaltenen Bilds umfasst;

wobei die Datenübertragungseinrichtung (17) dazu eingerichtet ist, die durch die Komprimierungseinrichtung (24) komprimierten Daten bei einer Mehrzahl von Übertragungsraten an die sich außerhalb des Körpers befindende Einheit (4) zu übertragen;

wobei die Bildaufnahmeeinheit ferner umfasst:

eine Einrichtung (37) zum Speichern einer komprimierten Datengröße zum Speichern der durch die Komprimierungseinrichtung (24) komprimierten Datengröße;

eine Einrichtung (39) zum Berechnen einer Differenz einer komprimierten Datengröße zum Berechnen der Differenz zwischen der vorhergehenden komprimierten Datengröße, die in der Einrichtung (39) zum Speichern einer komprimierten Datengröße gespeichert ist, und der gegenwärtigen komprimierten Datengröße; und

wobei die Bestimmungseinrichtung (17) dazu eingerichtet ist, die Differenz der komprimierten Datengröße mit einem vorbestimmten Schwellwert zu vergleichen und zu bestimmen, ob das Bild gültig ist oder nicht, und

wobei die Datenübertragungseinrichtung (17) dazu eingerichtet ist, eine Datenübertragungsrate gemäß dem durch die Bestimmungseinrichtung (39) ausgegebenen Bestimmungsergebnis zu steuern.

17. Endoskopbildaufnahmegerät (1) gemäß Anspruch 16, wobei die Bildaufnahmeeinheit (3) eine Befehlsempfangseinrichtung (17, 19) zum Empfangen einer Mehrzahl von Befehlsarten von der sich außerhalb des Körpers befindenden Einheit (4) umfasst und

die Datenübertragungseinrichtung (17) dazu eingerichtet ist, eine Datenübertragungsrate basierend auf dem durch die Befehlsempfangseinrichtung (17, 19) empfangenen Befehl zu steuern und die Steuerung der Datenübertragungsrate durch die Bestimmungseinrichtung (39) basierend auf einem durch die Befehlsempfangseinrichtung (17, 19) empfangenen anderen Befehl außer Kraft zu setzen.

18. Endoskopbildaufnahmegerät (1) gemäß Anspruch 1, wobei die Bildaufnahmeeinheit (3) ferner umfasst:

eine Speichereinrichtung (4) zum Speichern des durch die Bildaufnahmeeinrichtung (3) erhaltenen Bilds;

wobei die Verarbeitungseinrichtung (15) dazu eingerichtet ist, das Bild aus der Speichereinrichtung (14) auszulesen und eine vorbestimmte Verarbeitung durchzuführen,

wobei, beim Speichern des Bilds, die Speichereinrichtung (14) dazu eingerichtet ist, mit dem Hochgeschwindigkeitstakt zu arbeiten, der dem der Bildaufnahmeeinrichtung (3) ähnelt und, beim Verarbeiten des Bilds, die Speichereinrichtung (14) bei dem Niedriggeschwindigkeitstakt arbeitet, der dem der Verarbeitungseinrichtung (15) ähnelt.

## Revendications

1. Appareil de capture d'image endoscopique (1) comportant une unité de capture d'image (3) destinée à capturer une image du corps et à transmettre l'image par radio vers une unité extracorporelle (4), l'unité de capture d'image (3) comprenant :

un moyen de capture d'image (13) destiné à capturer l'image ;
un moyen de traitement de données (15) destiné à réaliser le traitement pour réduire la quantité de données de l'image obtenue par le moyen de capture d'image (13) à une pluralité de rapports ;
un moyen de transmission de données (17, 19) destiné à transmettre les données traitées par le moyen de traitement de données (15) vers l'unité extracorporelle (4) ;
un moyen de détection de quantité de caractéristiques (25, 26) destiné à détecter une quantité prédéterminée de caractéristiques sur la base de l'image ; et
un moyen de détermination (25, 26) destiné à déterminer sur la base de la quantité de caractéristiques si oui ou non l'image est valide ;
dans lequel le moyen de traitement de données (15) est adapté pour commander un rapport de réduction de la quantité de données conformément au résultat de détermination délivré en sortie par le moyen de détermination (25, 26) ;
dans lequel le moyen de détection de quantité de caractéristiques (25, 26) comprend un moyen de détermination d'histogramme (47, 61 à 66) destiné à détecter la quantité de caractéristiques, un histogramme de teintes et un histogramme de saturation de l'image à partir des données image, et
dans lequel le moyen de détermination d'histogramme (47, 61 à 66) délivre en sortie un signal de détection de répartition de couleurs indiquant que l'image est valide, lorsque l'histogramme de teintes montre qu'une distance entre deux valeurs de teinte crêtes à une valeur de seuil ou plus se situe à l'intérieur d'une plage prédéterminée, ou lorsque l'histogramme de saturation montre qu'une distance entre deux valeurs de saturation crêtes à une valeur de seuil ou plus se situe à l'intérieur d'une plage prédéterminée.

2. Appareil de capture d'image endoscopique (1) selon la revendication 1, dans lequel le moyen de détermination (25, 26) comprend :

un moyen de détermination d'invalidité (25) destiné à déterminer si l'image est valide ou invalide ; et
un moyen de détermination d'image cible (26) destiné à déterminer si oui ou non l'image est une image cible, dans lequel, sur la base de la combinaison des résultats de détermination à une pluralité d'étapes, l'image est déterminée comme étant valide et le résultat de détermination est délivré en sortie.

3. Appareil de capture d'image endoscopique (1) selon la revendication 1, dans lequel le moyen de détection de quantité de caractéristiques (26) comprend un moyen de détection de nombre de pixels (52) destiné à détecter le nombre de pixels présentant une couleur spécifique dans l'image en tant que quantité de caractéristiques, et le moyen de détermination (26) détermine que l'image est valide lorsque le nombre de pixels de couleur spécifique correspond à une valeur de seuil prédéterminée ou plus.

4. Appareil de capture d'image endoscopique (1) selon la revendication 1, dans lequel le moyen de détection de quantité de caractéristiques (26) comprend :

un moyen de détection de nombre de pixels (52) destiné à détecter le nombre de pixels présentant une couleur spécifique dans l'image ;
un moyen de mémorisation de nombre de pixels (56) destiné à mémoriser le nombre de pixels détecté ; et
un moyen de comparaison et de calcul de nombre de pixels (58) destiné à comparer et à calculer le nombre antérieur de pixels mémorisé dans le moyen de mémorisation de nombre de pixels (56) et le nombre actuel de pixels et destiné à délivrer en sortie la quantité de changement du nombre de pixels en tant que quantité de caractéristiques,
dans lequel le moyen de détermination (26) est adapté pour déterminer que l'image est valide lorsque la quantité de changement du nombre de pixels correspond à une valeur de seuil prédéterminée ou plus.

5. Appareil de capture d'image endoscopique (1) selon la revendication 1, dans lequel le moyen de détection de quantité de caractéristiques (26) comprend :

un moyen de comparaison et de calcul de répartition de couleurs (47, 48) destiné à comparer et à calculer l'histogramme de teintes et l'histogramme de saturation détectés et un histogramme de teintes et un histogramme de saturation prédéterminés et destiné à délivrer en sortie une erreur de l'histogramme de teintes et de l'histogramme de saturation en tant que quantité de caractéristiques, et
le moyen de détermination (26) est adapté pour déterminer que l'image valide lorsque l'erreur de l'histogramme de teintes et de l'histogramme de saturation correspond à une valeur de seuil prédéterminée ou moins.

6. Appareil de capture d'image endoscopique (1) selon la revendication 1, dans lequel le moyen de détection de quantité de caractéristiques (25) comprend un moyen de calcul de valeur moyenne de luminance (32) destiné à détecter une valeur moyenne des valeurs de luminance dans l'image en tant que quantité de caractéristiques, dans lequel le moyen de détermination (26) est adapté pour déterminer que l'image est valide lorsque la valeur moyenne de luminance se situe à l'intérieur d'une plage prédéterminée.

**7.** Appareil de capture d'image endoscopique (1) selon la revendication 1, dans lequel le moyen de détection de quantité de caractéristiques (25) comprend :

un moyen de calcul de valeur moyenne de luminance (32) destiné à détecter une valeur moyenne des valeurs de luminance dans l'image ;

un moyen de mémorisation de valeur moyenne de luminance (36) destiné à mémoriser la valeur moyenne de luminance ; et

un moyen de comparaison et de calcul de valeur moyenne de luminance (38) destiné à comparer et à calculer la valeur moyenne de luminance antérieure mémorisée dans le moyen de mémorisation de valeur moyenne de luminance (36) et la valeur moyenne de luminance actuelle et destiné à délivrer en sortie la quantité de changement de la valeur moyenne de luminance en tant que quantité de caractéristiques,

dans lequel le moyen de détermination (26) est adapté pour déterminer que l'image est valide lorsque la quantité de changement de la valeur moyenne de luminance correspond à une valeur de seuil prédéterminée ou plus.

**8.** Appareil de capture d'image endoscopique (1) selon la revendication 1, dans lequel le moyen de détection de quantité de caractéristiques (25) comprend :

un moyen de mémorisation d'image (14) destiné à mémoriser l'image capturée ; et

un moyen de calcul de différence de données image (41) destiné à calculer la différence entre les données image antérieures mémorisées dans le moyen de mémorisation d'image (14) et les données image actuelles et destiné à délivrer en sortie la différence calculée en tant que quantité de caractéristiques,

dans lequel le moyen de détermination (43) est adapté pour déterminer que l'image est valide lorsque la différence correspond à une valeur prédéterminée ou plus.

**9.** Appareil de capture d'image endoscopique (1) selon la revendication 1, dans lequel le moyen de traitement de données (15) comprend un moyen de réduction d'image (85) destiné à réduire les données image à une pluralité de rapports de réduction.

**10.** Appareil de capture d'image endoscopique (1) selon la revendication 1, dans lequel le moyen de traitement de données (15) comprend un moyen d'ajustement de longueur de bits (88) destiné à commuter la longueur de bits des données image à une pluralité de longueurs.

**11.** Appareil de capture d'image endoscopique (1) selon la revendication 1, dans lequel le moyen de traitement de données (15) comprend un moyen de découpe d'image (87) destiné à découper une partie des données image et destiné à délivrer en sortie l'image découpée.

**12.** Appareil de capture d'image endoscopique (1) selon la revendication 1, dans lequel le moyen de traitement de données (15) comprend un moyen de compression (86) destiné à compresser les données image à une pluralité de taux de compression.

**13.** Appareil de capture d'image endoscopique (1) selon la revendication 1, dans lequel l'unité de capture d'image (3) comprend un moyen de réception de commandes (17, 19) destiné à recevoir une pluralité de types de commandes depuis l'unité extracorporelle (4), et

le moyen de traitement de données (15) est adapté pour commander un rapport de réduction de la quantité de données sur la base de la commande reçue par le moyen de réception de commandes (17, 19), et pour invalider la commande du rapport de réduction de la quantité de données par le moyen de détermination (25, 26) sur la base d'une autre commande reçue par le moyen de réception de commandes (17, 19).

**14.** Appareil de capture d'image endoscopique (1) selon la revendication 1,

dans lequel le moyen de traitement de données (15) comprend

un moyen de compression (24) destiné à compresser l'image obtenue par le moyen de capture d'image (13) ;

dans lequel le moyen de transmission de données (17, 19) est adapté pour transmettre les données compressées par le moyen de compression (24) vers l'unité extracorporelle (4) à une pluralité de taux de transmission ; et

dans lequel le moyen de détermination (25, 26) est en outre adapté pour déterminer la validité de l'image en comparant la taille des données compressées par le moyen de compression (24) avec une valeur de seuil prédéterminée, et

dans lequel le moyen de transmission de données (17, 19) commande le taux de transmission de données conformément au résultat de détermination délivré en sortie par le moyen de détermination (25, 26).

**15.** Appareil de capture d'image endoscopique (1) selon la revendication 14, dans lequel l'unité de capture d'image (3) comprend un moyen de réception de commandes (17, 19) destiné à recevoir une pluralité de types de commandes depuis l'unité extracorporelle (4), et

le moyen de transmission de données (17) est adapté pour commander un taux de transmission de données sur la base de la commande reçue par le moyen de réception de commandes (17, 19), et invalide la commande du taux de transmission de données par le moyen de détermination (25, 26) sur la base d'une autre commande reçue par le moyen de réception de commandes (17, 19).

**16.** Appareil de capture d'image endoscopique (1) selon la revendication 1,

dans lequel le moyen de traitement de données (15) comprend un moyen de compression (24) destiné à compresser l'image obtenue par le moyen de capture d'image (13) ;

dans lequel le moyen de transmission de données (17) est adapté pour transmettre les données compressées par le moyen de compression (24) vers l'unité extracorporelle (4) à une pluralité de taux de transmission ;

dans lequel l'unité de capture d'image comprend en outre :

un moyen de mémorisation de taille de données compressées (37) destiné à mémoriser la taille des données compressées par le moyen de compression (24) ;

un moyen de calcul de différence de taille de données compressées (39) destiné à calculer la différence entre la taille des données compressées antérieure dans le moyen de mémorisation de taille de données compressées (39) et la taille des données compressées actuelle ; et

dans lequel le moyen de détermination (17) est adapté pour comparer la différence dans la taille des données compressées avec une valeur de seuil prédéterminée et pour déterminer si oui ou non l'image est valide, et

dans lequel le moyen de transmission de données (17) est adapté pour commander un taux de transmission de données conformément au résultat de détermination délivré en sortie par le moyen de détermination (39).

**17.** Appareil de capture d'image endoscopique selon la revendication 16, dans lequel l'unité de capture d'image (3) comprend un moyen de réception de commandes (17, 19) destiné à recevoir une pluralité de types de commandes depuis l'unité extracorporelle (4), et

le moyen de transmission de données (17) commande un taux de transmission de données sur la base de la commande reçue par le moyen de réception de commandes (17, 19), et invalide la commande du taux de transmission de données par le moyen de détermination (39) sur la base d'une autre commande reçue par le moyen de réception de commandes (17, 19).

**18.** Appareil de capture d'image endoscopique (1) selon la revendication 1, dans lequel l'unité de capture d'image (3) comprend en outre :

un moyen de mémorisation (4) destiné à mémoriser l'image obtenue par le moyen de capture d'image (3) ;

dans lequel le moyen de traitement (15) est adapté pour lire l'image à partir du moyen de mémorisation (14) et pour réaliser un traitement prédéterminé,

dans lequel, sur mémorisation de l'image, le moyen de mémorisation (14) est adapté pour fonctionner à une vitesse d'horloge élevée similaire à celle du moyen de capture d'image (3) et, sur traitement de l'image, le moyen de mémorisation (14) fonctionne à une faible vitesse d'horloge similaire à celle du moyen de traitement (15).

# FIG.1

EP 1 897 484 B1

# FIG.2

CONTROL IMAGE PICK-UP OPERATION

CONTROL BLOCK — 19

COMMAND

CONTROL COMMUNICATION

3

BATTERY — 21

DETECT INVALID IMAGE

DETECT AFFECTED PART

CONTROL PROCESSING USER CONTROL

IMAGE DATA

PROCESSING BLOCK

PROCESSED DATA

COMMUNICATION BLOCK

11 12

13

IMAGE MEMORY — 14

15

DATA MEMORY

17

16

CONTROL MEMORY CLOCK

COMMUNICATION CLOCK

20 — CLOCK SELECTOR

18

22

PROCESSING CLOCK

FREQUENCY-DIVIDING BLOCK

IMAGE CLOCK

EP 1 897 484 B1

# FIG.3

| IMAGE PICK-UP START PULSE | ∏ | | | ∏ |
|---|---|---|---|---|

| OPERATION OF IMAGE PICK-UP BLOCK | PICK-UP IMAGE | | STOP | | PICK-UP IMAGE |
|---|---|---|---|---|---|

S1

| OPERATION OF IMAGE MEMORY | STORE IMAGE | READ | STOP | | STORE IMAGE |
|---|---|---|---|---|---|

S2

| OPERATION OF PROCESSING BLOCK | STOP | PROCESS | STOP |
|---|---|---|---|

S3

| OPERATION OF DATA MEMORY | STOP | STORE PROCESSING RESULT | READ | STOP |
|---|---|---|---|---|

S4

| OPERATION OF COMMUNICATION BLOCK | STOP | TRANSMIT | STOP |
|---|---|---|---|

| PICK-UP IMAGE | IMAGE PROCESSING | TRANSMIT |
|---|---|---|

EP 1 897 484 B1

# FIG.4

```
IMAGE                    ┌──────────────┐ ┌24         COMPRESSED DATA
DATA    ─────────┬──────▶│ COMPRESSING  │──────────▶ (TO DATA MEMORY)
                 │        │    BLOCK     │
                 │        └──────────────┘
                 │                │
                 │         COMPRESSING
                 │            SIZE
                 │                ▼
                 │        ┌──────────────┐ ┌25        INVALID IMAGE
       15        ├───────▶│ INVALID IMAGE│──────────▶ DETECTING SIGNAL
                 │        │  DETECTING   │            (TO CONTROL BLOCK)
                 │        │    BLOCK     │
                 │        └──────────────┘
                 │
                 │        ┌──────────────┐ ┌26
                 │        │   AFFECTED   │            AFFECTED PART
                 └───────▶│     PART     │──────────▶ DETECTING SIGNAL
                          │  DETECTING   │            (TO CONTROL BLOCK)
                          │    BLOCK     │
                          └──────────────┘
```

23

# FIG.5

EP 1 897 484 B1

# FIG.6

LUMINANCE RANGE DETECTING BLOCK **27**

IMAGE DATA →

BEYOND-LUMINANCE-RANGE DETECTING SIGNAL

**30**

INVALID IMAGE DETECTING SIGNAL

AVERAGE LUMINANCE VALUE

IMAGE CHANGE DETECTING BLOCK **28**

IMAGE NO-CHANGE DETECTING SIGNAL

IMAGE COMPRESSING SIZE →

**25**

IMAGE COMPRESSING SIZE COMPARING BLOCK **29**

SIGNAL FOR DETECTING BEYOND-RANGE OF IMAGE COMPRESSING SIZE

(IMAGE COMPRESSING SIZE < Th_size)

EP 1 897 484 B1

# FIG.7

IMAGE DATA → **LUMINANCE VALUE INTEGRATING BLOCK** (31) → LUMINANCE VALUE INTEGRATED VALUE → **MULTIPLYING BLOCK** (32) → AVERAGE LUMINANCE VALUE Yav

1/NUMBER OF PIXELS →

**BLACK LEVEL THRESHOLD COMPARING BLOCK** (33)
(Yav < Th_Black)

**WHITE LEVEL THRESHOLD COMPARING BLOCK** (34)
(Yav > Th_White)

(35) → SIGNAL FOR DETECTING BEYOND-LUMINANCE-RANGE

(27)

AVERAGE LUMINANCE VALUE Yav

EP 1 897 484 B1

# FIG.8

# FIG.9

IMAGE
DATA $\longrightarrow$

IMAGE
DIFFERENCE
CALCULATING
BLOCK

*41*

DIFFERENCE
IMAGE $\longrightarrow$

DIFFERENCE
INTEGRATING
BLOCK

*42*

DIFFERENCE
INTEGRATED
VALUE
COMPARING
BLOCK

*43*

$\longrightarrow$ IMAGE NO-CHANGE
DETECTING SIGNAL

(INTEGRATED VALUE
OF DIFFERENCE<Th)

PREVIOUS-FRAME
IMAGE DATA

IMAGE
MEMORY

*14*

*28*

EP 1 897 484 B1

# FIG.10

IMAGE DATA
(R, G, B)

46
SPECIFIC COLOR
DETECTING
BLOCK

SPECIFIC-COLOR
DETECTING SIGNAL

NUMBER OF
SPECIFIC-COLOR
PIXELS

48
SPECIFIC-COLOR
CHANGE
DETECTING BLOCK

SPECIFIC-COLOR CHANGE
DETECTING SIGNAL

47
COLOR
DISTRIBUTION
CHARACTERISTIC
DETECTING BLOCK

COLOR DISTRIBUTION
CHARACTERISTIC
DETECTING SIGNAL

49

AFFECTED PART
DETECTING SIGNAL

26

# FIG.11

IMAGE DATA
(R, G, B) →

**51** — IMAGE DATA COMPARING BLOCK

$ThR\_Min < R < ThR\_Max$
$ThG\_Min < G < ThG\_Max$
$ThB\_Min < B < ThB\_Max$

**52** — SPECIFIC-COLOR PIXEL NUMBER COUNTING BLOCK

**46**

→ NUMBER OF SPECIFIC-COLOR PIXELS

**53** — SPECIFIC-COLOR PIXEL NUMBER COMPARING BLOCK

→ SPECIFIC-COLOR DETECTING SIGNAL

(NUMBER OF SPECIFIC-COLOR PIXELS > Th_Num)

EP 1 897 484 B1

# FIG.12

EP 1 897 484 B1

NUMBER OF
SPECIFIC-COLOR
PIXELS
→ **56** BLOCK FOR HOLDING NUMBER OF SPECIFIC-COLOR PIXELS IN PREVIOUS FRAME
→ **57** BLOCK FOR CALCULATING DIFFERENCE IN NUMBERS OF SPECIFIC-COLOR PIXELS
→ **58** BLOCK FOR COMPARING DIFFERENCE IN NUMBERS OF SPECIFIC-COLOR PIXELS
→ SPECIFIC-COLOR CHANGE DETECTING SIGNAL

(DIFFERENCE OF
SPECIFIC-COLOR PIXELS
>Th_Dif)

48

# FIG.13

EP 1 897 484 B1

IMAGE DATA (R, G, B) → **COLOR SPACE CONVERTING BLOCK** (61)

HUE → **HUE HISTOGRAM CALCULATING BLOCK** (62) → HUE HISTOGRAM → **HUE DISTRIBUTION CHARACTERISTIC DETECTING BLOCK** (64) → HUE DISTRIBUTION CHARACTERISTIC DETECTING SIGNAL

SATURATION → **SATURATION HISTOGRAM CALCULATING BLOCK** (63) → SATURATION HISTOGRAM → **SATURATION DISTRIBUTION CHARACTERISTIC DETECTING BLOCK** (65) → SATURATION DISTRIBUTION CHARACTERISTIC DETECTING SIGNAL

(66) → COLOR DISTRIBUTION DETECTING SIGNAL

47

# FIG.14A

FREQUENCY

HUE

# FIG.14B

FREQUENCY

SATURATION

# FIG.14C

FREQUENCY

HUE

# FIG.14D

FREQUENCY

SATURATION

# FIG.14E

FREQUENCY

HUE

# FIG.14F

FREQUENCY

SATURATION

# FIG.15

IMAGE DATA (R, G, B)

61

71 — Max VALUE DETECTING BLOCK

Max VALUE

Max_RGB

72 — Min VALUE DETECTING BLOCK

Min VALUE

73 — SATURATION CALCULATING BLOCK → SATURATION (S)

74 — HUE CALCULATING BLOCK → HUE (H)

# FIG.16

HUE VALUE →

62

76 — ADDRESS

DATA INPUT    DATA OUTPUT

HISTOGRAM MEMORY

→ HISTOGRAM RESULT

"1" →

77 — ADDER

EP 1 897 484 B1

# FIG.17A

# FIG.17B

# FIG.18

EP 1 897 484 B1

HISTOGRAM VALUE → HISTOGRAM VALUE COMPARING BLOCK *81*
(Hist>Th_Hist)

HUE VALUE →

EN / D Q / LATCH *82a*

EN / D Q / LATCH *82b*

BLOCK FOR DETECTING DIFFERENCE IN HUE VALUES *83*

BLOCK FOR COMPARING DIFFERENCE IN HUE VALUE *84*

→ HUE DISTRIBUTION CHARACTERISTIC DETECTING SIGNAL

*64*

(Th_DIF1<DIFFERENCE<Th_DIF2)

# FIG.19A

FREQUENCY

HUE

# FIG.19B

DIFFERENCE BETWEEN
PEAK HUE VALUES

PEAK HUE VALUE 1
IS LATCHED

PEAK HUE VALUE 2
IS LATCHED

HUE

# FIG.20

USER
CONTROL
SIGNAL

IMAGE
DATA

IMAGE SIZE
REDUCING
BLOCK          85

COMPRESSING
BLOCK          86

COMPRESSED DATA
(TO DATA MEMORY)

INVALID
IMAGE
DETECTING
SIGNAL

INVALID IMAGE
DETECTING
BLOCK          25

AFFECTED
PART
DETECTING
BLOCK          26

AFFECTED PART
DETECTING
SIGNAL

15

EP 1 897 484 B1

## FIG.21

INVALID IMAGE
DETECTING SIGNAL

AFFECTED PART
DETECTING SIGNAL

OPERATION OF
IMAGE SIZE
REDUCING BLOCK — SMALL IMAGE SIZE — MIDDLE IMAGE SIZE — LARGE IMAGE SIZE

OPERATION OF
COMPRESSING
BLOCK — HIGH COMPRESSING RATE — MIDDLE COMPRESSING RATE — LOW COMPRESSING RATE

STATUS OF
IMAGE PICK-UP UNIT — INVALID IMAGE — IMAGE AT NORMAL PART — IMAGE AT AFFECTED PART

# FIG.22A

USER CONTROL SIGNAL

AFFECTED PART DETECTING SIGNAL

INVALID IMAGE DETECTING SIGNAL

85

IMAGE DATA → | IMAGE CUT-OUT BLOCK (87) | → | SELECTOR (90a) | → | BIT-LENGTH REDUCING BLOCK (88) | → | SELECTOR (90b) | → | IMAGE REDUCING BLOCK (89) | → | SELECTOR (90c) | → SIZE-REDUCED IMAGE

# FIG.22B

| | IMAGE CUT-OFF BLOCK | BIT-LENGTH REDUCING BLOCK | IMAGE REDUCING BLOCK |
|---|---|---|---|
| INVALID IMAGE | ON | ON | ON |
| PICK UP IMAGE OF NORMAL PART | OFF | OFF | ON |
| PICK UP IMAGE OF AFFECTED PART | OFF | OFF | OFF |

EP 1 897 484 B1

# FIG.23

# FIG.24

# FIG.25

EP 1 897 484 B1

**EP 1 897 484 B1**

## REFERENCES CITED IN THE DESCRIPTION

**Patent documents cited in the description**

- JP 2002508201 A

- EP 1492352 A2